# EUROPEAN PATENT APPLICATION

(11) **EP 1 780 542 A1**
(43) Date of publication of application: **02.05.2007**
(21) Application number: 05765312.3
(22) Date of filing: 29.06.2005
(51) Int. Cl.: G01N 33/53, C12Q 1/37, C12Q 1/48, G01N 33/573

(54) **INDICATOR AGENT FOR NONINFLAMMATORY STRESS RESPONSE AND USE THEREOF**

(30) Priority: 30.06.2004 JP 2004193712; 31.01.2005 JP 2005023978
(71) Applicant: Sekiyama, Atsuo, Suita-shi, Osaka 5640063 (JP)
(72) Inventor: Sekiyama, Atsuo, Suita-shi, Osaka 5640063 (JP)
(74) Representative: Duckworth, Timothy John
(86) International application number: PCT/JP2005/011933
(87) International publication number: WO 2006/003927

(57) **Abstract**

A system enabling the molecular biological visualization and quantitative detection of events in a stress-exposed living organism and a means enabling the management of stress are provided. An indicator agent for non-inflammatory stress responses mediated by superoxide, which comprises IL-18, a visualizing agent for non-inflammatory stress responses for detecting the aforementioned indicator agent, a method of measuring the degree of non-inflammatory stress, which comprises using the aforementioned visualizing agent, a method of preventing, ameliorating or predicting a change in immune status based on a non-inflammatory stress response, which comprises applying the aforementioned visualizing agent to an animal, and a therapeutic agent for a change in immune status based on a non-inflammatory stress response for reducing the amount or activity of the aforementioned indicator agent.

## Description

### Technical Field

The present invention relates to an indicator agent for non-inflammatory stress responses and use thereof. More specifically, the present invention is directed to elucidating stress molecular biologically, thereby contribute to scientific development in the field of mental health.

### Background Art

Mental and/or physical stress influences host defense, including the nervous, endocrine and immune systems (see non-patent documents 1 and 2). Various cytokines (for example, IL-1β, IL-6, TNF-α and the like) undergo upregulation by stress (see non-patent documents 1 and 3). This suggests the possible involvement of cytokines in the interference of host defense (see non-patent document 4). However, the molecular mechanisms by which stress induces cytokines that damage host defense remain to be understood fully.

Interleukin-18 (IL-18) is a cytokine discovered as an interferon-γ (IFN-γ) inducing factor (see non-patent document 5 and patent document 1). IL-18 has a broad range of biological activities, including the induction of Fas ligands, the elevation of the cytolytic activity of T cells (see non-patent document 6) and the production of IL-4 and IL-13 (see non-patent document 7) (see non-patent document 8). IL-18 activates Toll-like receptor 2 (see non-patent document 9) and activates myelodifferentiation protein (Myd)-88 (see non-patent document 10). The activation thereof is essential to the induction of IL-6 (see non-patent document 11). Therefore, IL-18 is involved in the production of both the cytokines Th1 and Th2 (see non-patent document 12).

IL-18 is produced as a 24-kD precursor protein and processed into the 18-kD mature active type by IL-1β converting enzyme (ICE, or also referred to as caspase-1) (see non-patent document 13). Caspase-1 is induced as inactive type precursor protein procaspase-1 and activated by caspase-11 (see non-patent document 14). The expression of caspase-11 mRNA requires the trans-activation of NF-κB (see non-patent document 15), and this activation is reported to be mediated by P38MAP kinase (see non-patent document 16). The induction of caspase-11 mRNA by LPS (lipopolysaccharide) and the subsequent activation of caspase-1 are reported to be suppressed by SB203580, which is a P38MAP kinase inhibitor, in glyoma cell line C6 (see non-patent document 17).

Recent research has reported that IL-18 mRNA is expressed in the adrenal gland in response to adrenocorticotrophic hormone (ACTH) and cold stress (see non-patent document 18). Also, it has been reported that in the adrenal gland and immunocytes, IL-18 mRNA uses different promoters (see non-patent document 19). However, the induction of the mature type of IL-18 is not shown in either of the aforementioned two studies. On the other hand, in psychiatric patients, elevations of IL-18 in plasma have been reported (see non-patent document 20).

In modern society, humans are working and living while being exposed to various forms of stress. Generally, there are individual differences in how stress is felt, with no definite index available for the presence or absence and intensity of stress. Research to date has not shown whether or not stress results in increases in cytokines such as IL-18, and whether or not IL-18 plays a role in the disorder of host defense induced by stress.
Patent document 1: Japanese Patent Unexamined Publication No. HEI-8-193098
Non-patent document 1: Dugue, B. et al. Scand. J. Clin. Invest. 53, 555-561 (1993)
Non-patent document 2: Kiecolt-Glaser, J.K. et al. Proc. Natl. Acad. Sci. USA 93, 3043-3047 (1996)
Non-patent document 3: Endocrinology 133, 2523-2530 (1993)
Non-patent document 4: Schubert, C. et al. Psychosom. Med. 61, 876-882 (1999)
Non-patent document 5: Zhou, D. et al. Nature 378, 88-91 (1995)
Non-patent document 6: Nakanishi, K. et al. Annu. Rev. Immunol. 19 423-474 (2001)
Non-patent document 7: Hoshino, T. et al. J. Immunol. 162, 5070-5077 (1999)
Non-patent document 8: Dinarello, C.A. et al. J. Leukoc. Biol. 63, 658-664 (1998)
Non-patent document 9: Blease, K. et al. Inflamm. Res. 50, 552-560 (2001)
Non-patent document 10: Adachi, O. et al. Immunity 9, 143-150 (1998)
Non-patent document 11: Takeuchi, O. et al. J. Immunol. 165, 5392-5396 (2000)
Non-patent document 12: Hoshino, T. et al. J. Immunol. 166, 7014-7018 (2001)
Non-patent document 13: Gu, Y. et al. Science 275, 206-209 (1997)
Non-patent document 14: Wang, S. et al. Cell 92, 501-509 (1998)
Non-patent document 15: Schauvliege, R. et al. J. Biol. Chem. 277, 41624-41630 (2002)
Non-patent document 16: Vanden Berghe, W. et al. J. Biol. Chem. 273, 3285-3290 (1998)
Non-patent document 17: Hur, J. et al. FEBS Lett. 507, 157-162 (2001)
Non-patent document 18: Conti, B. et al. J. Biol. Chem. 272, 2035-2037 (1997)
Non-patent document 19: Suganuma, S. et al. J. Immunol. 165, 6287-6292 (2000)
Non-patent document 20: Kokai, M. et al. J. Immunother. 25, 68-71 (2002)

### Disclosure of the Invention

### Problems to Be Solved by the Invention

It is an object of the present invention to provide a system capable of molecular biologically visualizing and quantitatively detecting events in an living organism exposed to stress. It is another object of the present invention to provide a means that enables the management and avoidance of physical disorders due to stress.

### Means of Solving the Problems

The present inventor conducted research with a focus on the processing of IL-18 precursor protein and the release of mature type IL-18 into plasma in examining the expression of cytokines in mice undergoing restraint stress, found that an cascade in a living organism to activate IL-18 is induced by stress, and accomplished the present invention.

Accordingly, the present invention relates to:
[1] an indicator agent for superoxide-mediated non-inflammatory stress responses, which comprises an ingredient involved in the non-inflammatory stress cascade,
[2] the indicator agent described in [1] above, wherein the ingredient involved in the non-inflammatory stress cascade contains 1 or 2 or more proteins selected from the group consisting of IL-18, active type IL-18, P38MAP kinase, phosphorylated P38MAP kinase, caspase 11, caspase 1, and activated caspase 1,
[3] the indicator agent described in [1] above, wherein the ingredient involved in the non-inflammatory stress cascade is IL-18,
[4] the indicator agent described in [3] above, wherein the IL-18 is active type IL-18 present in blood,
[5] the indicator agent described in [3] or [4] above, which further comprises P38MAP kinase,
[6] the indicator agent described in [5] above, wherein the P38MAP kinase is phosphorylated kinase,
[7] the indicator agent described in [6] above, wherein the P38MAP kinase is derived from a mouse, and the phosphorylation site of the kinase is Thr180 or Tyr182,
[8] the indicator agent described in [6] above, wherein the P38MAP kinase is derived from a human, and the phosphorylation site of the kinase is Thr185 or Tyr187,
[9] the indicator agent described in any of [3] to [8] above, which further comprises caspase-11,
[10] the indicator agent described in any of [3] to [9] above, which further comprises caspase-1,
[11] the indicator agent described in [10] above, wherein the caspase-1 is activated caspase-1,
[12] the indicator agent described in any of [3] to [11] above, wherein the non-inflammatory stress is mental stress,
[13] the indicator agent described in [12] above, wherein the mental stress is restraint stress,
[14] a visualizing agent for non-inflammatory stress responses for detecting the indicator agent described in any of [1] to [13] above,
[15] the visualizing agent described in [14] above, which comprises an IL-18 antibody,
[16] the visualizing agent described in [15] above, wherein the IL-18 antibody specifically recognizes active type IL-18,
[17] the visualizing agent described in [15] or [16] above, which further comprises a P38MAP kinase antibody,
[18] the visualizing agent described in [17] above, wherein the P38MAP kinase antibody is an anti-phosphorylated P38MAP kinase antibody,
[19] the visualizing agent described in [18] above, wherein the anti-phosphorylated P38MAP kinase antibody recognizes a P38MAP kinase having phosphorylated Thr180 or Tyr182, or a P38MAP kinase having phosphorylated Thr185 or Tyr187,
[20] the visualizing agent described in any of [15] to [19] above, which further comprises a caspase-11 antibody,
[21] the visualizing agent described in any of [15] to [20] above, which further comprises a caspase-1 antibody or a caspase 1 substrate,
[22] the visualizing agent described in [21] above, wherein the caspase-1 antibody specifically recognizes activated caspase-1,
[23] a method of measuring the degree of non-inflammatory stress, which comprises measuring the amount or activity of the indicator agent described in any of [1] to [13] above,
[24] the method described in [23] above, wherein the indicator agent is IL-18 or active type IL-18.
[25] the method described in [23] or [24] above, which comprises using the visualizing agent described in any of [14] to [22] above,
[26] the method described in any of [23] to [25] above, wherein the non-inflammatory stress is mental stress,
[27] the method described in [26] above, wherein the mental stress is restraint stress,
[28] an assay kit for non-inflammatory stress, which comprises the visualizing agent described in any of [14] to [22] above.
[29] the kit described in [28] above, wherein the visualizing agent comprises an IL-18 antibody,
[30] the kit described in [29] above, wherein the IL-18 antibody recognizes active type IL-18,
[31] a method of preventing, ameliorating or predicting a change in immune status based on a non-inflammatory stress response, which comprises applying the visualizing agent described in any of [14] to [22] above to an animal,
[32] the preventing, ameliorating or predicting method described in [31] above, wherein the animal is a vertebral animal other than a human,
[33] a therapeutic agent for a change in immune status based on a non-inflammatory stress response for reducing the amount or activity of the indicator agent described in any of [1] to [13] above,
[34] the therapeutic agent described in [33] above, wherein the active ingredient of the therapeutic agent is an IL-18 antibody or an anti-IL-18 receptor antibody,
[35] the therapeutic agent described in [34] above, wherein the active ingredient is an IL-18 antibody, and the IL-18 antibody binds specifically to active type IL-18,
[36] the therapeutic agent described in [33] above, wherein the active ingredient of the therapeutic agent is superoxide dismutase or vitamin E,
[37] a method of screening for a substance having a therapeutic effect, ameliorating effect or prophylactic effect on a disease that occurs, exacerbates or recurs due to non-inflammatory stress, which comprises measuring the amount or activity of the indicator agent described in any of [1] to [13] above,
[38] the method described in [37] above, wherein the indicator agent is IL-18 or active type IL-18,
[39] the method described in [37] or [38] above, wherein the disease is systemic lupus erythematosus, lupus nephritis, atopic dermatitis, multiple sclerosis, bronchial asthma, psoriasis, cerebral ischemia, cerebral degeneration, encephalitis, articular rheumatism, emmeniopathy, endometriosis or sexual desire reduction, and
[40] a method of screening for a substance having a suppressive effect or preventive effect on oxidative stress, which comprises measuring the amount or activity of the indicator agent described in any of [1] to [13] above.

### EFFECT OF THE INVENTION

According to the indicator agent of the present invention, the levels of IL-18 components and a cascade having P38MAP kinase as the starting point, mediated by superoxide produced due to non-inflammatory stress (hereinafter referred to as the stress cascade) are clarified, and this is helpful as an index not only for the degree of non-inflammatory stress to which each living organism undergoes, but also for the evaluation or prediction of the risk of collapse of host defense.

Also, according to the indicator agent of the present invention, because it contains ingredients involved in the stress cascade, it can be utilized for screening of a substance that reduces these ingredients and activities, and is helpful in developing pharmaceuticals and foods with health claims (foods for specified health uses and foods with nutrient function claims) comprising such a substance as an active ingredient.

According to the visualizing agent of the present invention, the levels of IL-18 and the stress cascade can be quantified, and it is possible to open a new way to controlling non-inflammatory stress, which has been difficult to control.

According to the method of the present invention of measuring the degree of non-inflammatory stress, the degree of stress to which each living organism undergoes can easily be quantified, and it is particularly useful for taking measures considering laborers' mental health.

According to the method of the present invention of preventing, ameliorating or predicting a change in immune status based on a non-inflammatory stress response, by applying the method to animals such as domestic or companion animals, an effect is obtained of keeping animals in good immune status and rearing animals unlikely to contract disease in the field of animal husbandry or pet industry, in which artificial rearing is likely to lead to stress loading.

According to the therapeutic agent of the present invention for a change in immune status, it is helpful for the treatment of a disease that occurs, exacerbates or recurs due to non-inflammatory stress.

Because keratinocytes constitute the epidermis, the release of IL-18 is thought to have a significant effect on the inflammation and immunity of the skin. In fact, the present inventor confirmed that inflammation and immune reduction due to ultraviolet were less severe, and skin hardening and thickening did not occur in mice that do not express IL-18. Hence, according to the therapeutic agent of the present invention, abnormalities of the release of IL-18, and hence of the inflammation, immunological changes, and abnormal regulation of differentiation/growth of the skin can be prevented. This is a totally new idea; the therapeutic agent of the present invention is effective against skin disorders due to ultraviolet and the like, and atopic dermatitis, psoriasis, acne vulgaris, pemphigus, ichthyosis, and photosensitivity, which are postulated to be involved by IL-18, as well as against burns, radiation disorders of the skin, wounds and the like involving inflammatory changes in the skin in their pathology. Furthermore, use of the visualizing agent of the present invention is helpful in the elucidation of the pathology, drug innovation development, and therapeutic model development.

Microglia and astrocytes are distributed in the central nervous system, playing central roles in neural survival, inflammation in the central nervous system, and the body's defense system. Because IL-18 has effects such as causing inflammation when acting acutely, the therapeutic agent of the present invention is applicable to brain inflammation, neuronal loss, and brain insufficiencies involved by microglia or astrocytes. For example, the therapeutic agent of the present invention is applicable to diseases such as Alzheimer's disease, Alzheimer type senile dementia, diffuse Lewy body disease, Pick's disease, Binswanger's disease, Parkinson's disease, Parkinson syndrome, cerebral ischemia, cerebral ischemia/reperfusion disorders, carbon monoxide poisoning, thinner poisoning, neonatal hemorrhagic encephalopathy, hypoxic encephalopathy, hypertensive encephalopathy, epilepsy, multiple sclerosis, HIV encephalopathy, brain circulation disorders, and cerebrovascular disorders. Also, because astrocytes and microglia also occur in endocrine-related sites such as the hypothalamus and the pituitary, and are involved in nervous endocrine regulation, IL-18 from those glial cells is also involved in circadian rhythm abnormalities, appetite abnormalities, pituitary insufficiencies such as Addison's disease, acromegaly, and gonadal developmental disorders, thyroid functional impairment, obesity, and blood glucose control abnormalities, and secondarily in adrenal insufficiencies such as primary aldosteronism and Cushing's disease, gastrointestinal tract movement regulation and abnormalities in bone formation, and the like; with the therapeutic agent of the present invention, the process in which the above-described diseases and insufficiencies develop and exacerbate due to non-inflammatory stress can be avoided and controlled.

Macrophages occur everywhere in a living organism, playing a central role in body defense reactions and control of inflammation by migrating from blood vessels into tissue and the like. According to the therapeutic agent of the present invention, all inflammatory diseases mediated by macrophages and IL-18 with the involvement of non-inflammatory stress, autoimmune disease, and body defense system regulatory abnormalities, can be avoided and controlled.

It is known that synovial cells constitute articular capsules, and that particularly in articular rheumatism, inflammation involved by synovial cells and macrophages pathologically characterizes the condition. Also, if inflammation occurs in synovial cells due to arthritis and the like, joint functional disorders and limitations on the range of motion are caused by hypertrophic deformation. Although these phenomena had been suggested to involve cytokines, controlling cytokines downstream of the cytokine release cascade is subject to limitation as to inflammation control, with aggravation of inflammation due to rebound and the like have been problematic. According to the therapeutic agent of the present invention, the release of IL-18 not only from macrophages, but also from synovial membrane tissue, can be suppressed and avoided, and the subsequent mechanism for the release of cytokines and inflammation can be controlled. Thereby, the occurrence, recurrence, and exacerbation of diseases pathologically characterized by joint inflammation, such as articular rheumatism, gout, and arthritis, due to non-inflammatory stress such as cold or mental stress, insufficient sleep, bloodstream disorders, and physical weight loading, can be avoided and controlled by controlling the visualizing agent or indicator agent of the present invention.

Renal tubular epithelial cells play an important role in body fluid regulation; localized disturbance thereof results in renal insufficiency, body fluid regulation abnormalities, collapse of the potassium-sodium-chlorine ion balance, edema, glucose tolerance impairments such as diabetes mellitus, emaciation, convulsion, heart failure, pulmonary edema, hypoproteinemia, bleeding tendency and the like. According to the therapeutic agent of the present invention, the occurrence and exacerbation of diseases pathologically characterized by renal tubular epithelial cell loss and inflammation, due to infection, poisons, and anticancer agents that injure the renal tubules, such as cisplatin and carboplatin, or by an idiopathic mechanism, can be avoided and controlled.

Also, because an elevation of blood IL-18 causes changes in the blood levels of adrenocorticotrophic hormone (ACTH), follicle-stimulating hormone (FSH), and luteinizing hormone (LH), by controlling the release of blood IL-18 with the therapeutic agent of the present invention, the occurrence, recurrence, and exacerbation of diseases caused by pituitary insufficiencies, such as emmeniopathy, endometriosis, and sexual desire reduction, due to non-inflammatory stress such as cold or mental stress, insufficient sleep, bloodstream disorders, and physical weight loading, can be avoided and controlled by controlling the visualizing agent or indicator agent of the present invention.

### Brief Description of the Drawings

[Fig. 1A] A drawing showing plasma IL-18 and ACTH levels undergoing upregulation by restraint stress.
[Fig. 1B] A drawing showing that plasma IL-18 levels undergoing upregulation by restraint stress are suppressed by anti-ACTH antibody or dexamethasone.
[Fig. 1C] A drawing showing that IL-18 in the adrenal undergoing upregulation by restraint stress is suppressed by anti-ACTH antibody or dexamethasone.
[Fig. 1D] A drawing comparing IL-18 in plasma after administration of ACTH and IL-18 in plasma after restraint stress loading.
[Fig. 1E] A drawing comparing IL-18 in the adrenal after administration of ACTH and IL-18 in the adrenal after restraint stress loading.
[Fig. 1F] A drawing showing the results of an examination of IL-18 protein in the adrenal (lanes 1 to 3) and plasma (lanes 4 to 6) by Western blotting. Lanes 1 and 4 show the results obtained with control, lanes 2 and 5 show the results obtained after ACTH was administered, and lanes 3 and 6 show the results obtained after restraint stress was loaded.
[Fig. 2A] A drawing showing the results of an examination of procaspase-1 in the adrenal by Western blotting. Lane 1 shows the results obtained with control, lane 2 shows the results obtained after ACTH administration, and lane 3 shows the results obtained after restraint stress loading.
[Fig. 2B] A drawing showing that caspase-1 activity in the adrenal undergoing upregulation by restraint stress is suppressed by the caspase-1 inhibitor YVAD-CHO.
[Fig. 2C] A drawing showing that IL-18 in plasma undergoing upregulation by restraint stress is suppressed by YVAD-CHO.
[Fig. 2D] A drawing showing that IL-18 in the adrenal undergoing upregulation by restraint stress is not suppressed by YVAD-CHO.
[Fig. 2E] A drawing showing that caspase-1 activity in the adrenal undergoing upregulation by restraint stress is suppressed by the caspase-11 inhibitor Z-FA-fmk.
[Fig. 2F] A drawing showing that IL-18 activity in plasma undergoing upregulation by restraint stress is suppressed by the caspase-11 inhibitor Z-FA-fmk.
[Fig. 3A] A drawing showing that the caspase-11 precursor undergoing upregulation by restraint stress is suppressed by SB203580, which is a P38MAP kinase inhibitor.
[Fig. 3B] A drawing showing that caspase-1 activity induced by restraint stress is suppressed by SB203580.
[Fig. 3C] A drawing showing that IL-18 in plasma induced by restraint stress is suppressed by SB203580.
[Fig. 3D] A drawing showing the results of an examination of phosphorylated P38MAP kinase in the adrenal by Western blotting. Lane 1 shows the results obtained without stress, lane 2 shows the results obtained with stress loading, lane 3 shows the results obtained with the addition of YVAD-CHO, lane 4 shows the results obtained with the addition of Z-FA-fmk, and lane 5 shows the results obtained in the case of stress-loaded caspase-1 KO mice.
[Fig. 3E] A photograph showing the results of staining of the adrenal of a stress-loaded mouse using immunohistochemistry. In the overlapping region in the zona reticularis of adrenal cortex, the induction of IL-18 (panel 1), pro/caspase-1 (panel 2), pro/caspase-11 (panel 3), and activated (phosphorylated) P38MAP kinase (panel 4) is shown.
[Fig. 3F] A photograph showing the expression of activated (phosphorylated) P38MAP kinase in the presence of caspase inhibitor. Panel 1 shows the results obtained with the addition of Z-FA-fmk, and panel 2 shows the results obtained with the addition of YVAD-CHO.
[Fig. 4A] A drawing showing that activated (phosphorylated) P38MAP kinase undergoing upregulation by restraint stress is suppressed by SOD.
[Fig. 4B] A drawing showing that procaspase-11 undergoing upregulation by restraint stress is suppressed by SOD.
[Fig. 4C] A drawing showing that caspase-1 activity induced by restraint stress is suppressed by SOD.
[Fig. 4D] A drawing showing that IL-18 in plasma undergoing upregulation by restraint stress is suppressed by SOD.
[Fig. 4E] A drawing showing that IL-18 in the adrenal undergoing upregulation by restraint stress is not suppressed by SOD.
[Fig. 4F] A drawing showing that IL-18 in plasma undergoing upregulation by restraint stress is suppressed by DPI.
[Fig. 5A] A drawing showing plasma IL-6 levels in stress-loaded mice.
[Fig. 5B] A drawing showing that in stress-loaded wild type mice, plasma IL-6 levels undergoing upregulation are suppressed by caspase-1 inhibitor, SOD and anti-IL-18 antibody.
[Fig. 6] A drawing showing that in MRL/lpr mice, the exacerbation of lupus nephritis due to stress loading is suppressed by administration of anti-IL-6 antibody or anti-IL-18 receptor antibody.
[Fig. 7A] A drawing showing the effect of a suppressant of superoxide (SOD) on IL-18 release from keratinocytes with paraquat stimulation.
[Fig. 7B] A drawing showing the effect of a suppressant of superoxide (vitamin E) on IL-18 release from keratinocytes with paraquat stimulation.
[Fig. 7C] A drawing showing the effect of a suppressant of superoxide (SOD) on IL-18 release from keratinocytes with UV-B ray irradiation.
[Fig. 7D] A drawing showing the effect of a suppressant of superoxide (vitamin E) on IL-18 release from keratinocytes with UV-B ray irradiation.
[Fig. 8A] A drawing showing the effect of a suppressant of superoxide (SOD) on IL-18 release from microglia cells with paraquat stimulation.
[Fig. 8B] A drawing showing the effect of a suppressant of superoxide (vitamin E) on IL-18 release from microglia with paraquat stimulation.
[Fig. 9A] A drawing showing the effect of a suppressant of superoxide (SOD) on IL-18 release from astrocytes with paraquat stimulation.
[Fig. 9B] A drawing showing the effect of a suppressant of superoxide (vitamin E) on IL-18 release from astrocytes with paraquat stimulation.
[Fig. 10A] A drawing showing the effect of a suppressant of superoxide (SOD) on IL-18 release from macrophages with paraquat stimulation.
[Fig. 10B] A drawing showing the effect of a suppressant of superoxide (vitamin E) on IL-18 release from macrophages with paraquat stimulation.
[Fig. 11A] A drawing showing the effect of a suppressant of superoxide (SOD) on IL-18 release from synovial membrane tissue with paraquat stimulation.
[Fig. 11B] A drawing showing the effect of a suppressant of superoxide (vitamin E) on IL-18 release from synovial membrane tissue with paraquat stimulation.
[Fig. 12A] A drawing showing the effect of a suppressant of superoxide (SOD) on IL-18 release from renal tubular epithelial cells with paraquat stimulation.
[Fig. 12B] A drawing showing the effect of a suppressant of superoxide (vitamin E) on IL-18 release from renal tubular epithelial cells with paraquat stimulation.
[Fig. 13A] A drawing showing changes in plasma ACTH concentration caused by IL-18 administration.
[Fig. 13B] A drawing showing changes in plasma FSH concentration caused by IL-18 administration.
[Fig. 13C] A drawing showing changes in plasma LH concentration caused by IL-18 administration.

### Best Mode for Embodying the Invention

The present invention is based on the finding of a novel cascade mediated by superoxide induced by non-inflammatory stress (stress cascade), making it possible to visualize and manage non-inflammatory stress.

Abbreviations for amino acids, (poly)peptides, (poly)nucleotides and the like hereinafter used in the present description are based on the IUPAC-IUB rules [IUPAC-IUB Communication on Biological Nomenclature, Eur. J. Biochem., 138:9 (1984)], "Guideline for the Preparation of Descriptions etc. Including Base Sequences or Amino Acid Sequences" (edited by the Japan Patent Office), and abbreviations in common use in relevant fields.

"Proteins" or "(poly)peptides" as used herein include not only "proteins" or "(poly)peptides" shown by particular amino acid sequences (SEQ ID NO:2, 4, 6, 8 or 10), but also homologous forms (homologues and splice variants), mutants, derivatives, mature forms and amino acid modifications thereof and the like, as long as they are equivalent thereto in terms of biological function. Here, as examples of the homologues, proteins of other biological species such as mice and rats, corresponding to human proteins, can be mentioned, and these can be deductively identified from the base sequences of genes identified by HomoloGene (http://www.ncbi.nlm.nih.gov/HomoloGene/). Also, the mutants include naturally occurring allele mutants, non-naturally-occurring mutants, and mutants having an amino acid sequence altered by artificially deleting, substituting, adding, or inserting an amino acid. Note that as the above-described mutants, those having a homology of at least 70%, preferably 80%, more preferably 95%, still more preferably 97%, to mutation-free proteins or (poly)peptides, can be mentioned. Also, the amino acid modifications include naturally occurring amino acid modifications and non-naturally-occurring amino acid modifications, and specifically, phosphorylated forms of amino acids can be mentioned.

Accordingly, the term "IL-18 protein" or simply "IL-18" as used herein, unless otherwise specified by a sequence identification number, is intended to include human IL-18, which is shown by a particular amino acid sequence (SEQ ID NO:2), homologous forms, mutants, derivatives, mature forms and amino acid modifications thereof and the like. Specifically, human IL-18, which has the amino acid sequence shown by SEQ ID NO:2 (GenBank Accession No. NM_001562), mouse homologues and rat homologues thereof and the like are included.

Also, the term "P38MAP kinase protein" or simply "P38MAP kinase" as used herein, unless otherwise specified by a sequence identification number, is intended to include human P38MAP kinase, which is shown by a particular amino acid sequence (SEQ ID NO:4), or homologous forms, mutants, derivatives, mature forms and amino acid modifications thereof and the like. Specifically, human P38MAP kinase, which has the amino acid sequence shown by SEQ ID NO:4 (GenBank Accession No. NM_138957), mouse homologues and rat homologues thereof and the like are included.

Also, the term "caspase-11 protein" or simply "caspase-11" as used herein, unless otherwise specified by a sequence identification number, is intended to include mouse caspase-11, which is shown by a particular amino acid sequence (SEQ ID NO:6), and homologous forms, mutants, derivatives, mature forms and amino acid modifications thereof and the like. Specifically, mouse caspase-11, which has the amino acid sequence shown by SEQ ID NO:6 (GenBank Accession No. MMCASP11), human homologues and rat homologues thereof and the like are included.

Also, the term "caspase-1 protein" or simply "caspase-1" as used herein, unless otherwise specified by a sequence identification number, is intended to include human caspase-1, which is shown by a particular amino acid sequence (SEQ ID NO:8), homologous forms, mutants, derivatives, mature forms and amino acid modifications thereof and the like. Specifically, human caspase-1, which has the amino acid sequence shown by SEQ ID NO:8 (GenBank Accession No. NM_033292), mouse homologues and rat homologues thereof and the like are included.

Also, the term "IL-6 protein" or simply "IL-6" as used herein, unless otherwise specified by a sequence identification number, is intended to include human IL-6, which is shown by a particular amino acid sequence (SEQ ID NO:10), homologous forms, mutants, derivatives, mature forms and amino acid modifications thereof and the like. Specifically, human IL-6, which has the amino acid sequence shown by SEQ ID NO:10 (GenBank Accession No. NM_000600), mouse homologues and rat homologues thereof and the like are included.

In the present invention, "antibodies" include polyclonal antibodies, monoclonal antibodies, chimeric antibodies, single-stranded antibodies, or a part of the above-described antibodies having antigen bindability, such as Fab fragments and fragments produced with an Fab expression library.

Also, in the present invention, "a body defense system" denotes a defense system by immune system function, a defense system by nervous/endocrine system function, particularly a defense system by the cerebral cortex-hypothalamus-pituitary-adrenocortical system and the autonomic nervous system, and a host defense system by the nervous, endocrine and immune systems mediated by cytokines.

"Non-inflammatory stress" as used herein refers to stress excluding inflammatory stress due to infection, tumors and the like, includes what is called oxidative stress, which is mediated by superoxide, and is hereinafter sometimes abbreviated as "stress". As the non-inflammatory stress, specifically, physical stress due to high temperature, low temperature, high pressure, low pressure, noise, radiation, ultraviolet and the like, hypoxia, chemical stress due to harmful chemical substances such as heavy metals and arsenic, and mental stress due to anger, anxiety, fear, tension, restraint and the like, can be mentioned as examples, and in the present invention, physical stress, chemical stress, or mental stress is preferable; as the mental stress, restraint stress is more preferable, and restraint stress for a long time is particularly preferable. Here, a long time, not generically defined because of organism and individual differences, can be exemplified by 6 hours or more in the case of an animal. As the physical stress, stress due to ultraviolet irradiation is preferable; as the chemical stress, stress due to harmful chemical substance exposure, particularly stress due to paraquat exposure, is preferable.

"Non-inflammatory stress response" as used herein refers to an in vivo reaction of a living organism exposed to non-inflammatory stress to the stress.

"Superoxide" as used herein refers to a kind of active oxygen produced in a living organism, represented by superoxide anions or radicals, and is known to be eliminated by superoxide dismutase (SOD), or to react with other substances, in the body, and is used in the present invention with a meaning including the metabolites of superoxide.

The "indicator agent" of the present invention serves as an index for non-inflammatory stress responses mediated by superoxide, and has been developed by the discovery of "the cytokine cascade in non-inflammatory stress (referred to as the stress cascade)", which was for the first time demonstrated by the present inventor on the basis of the findings described below.

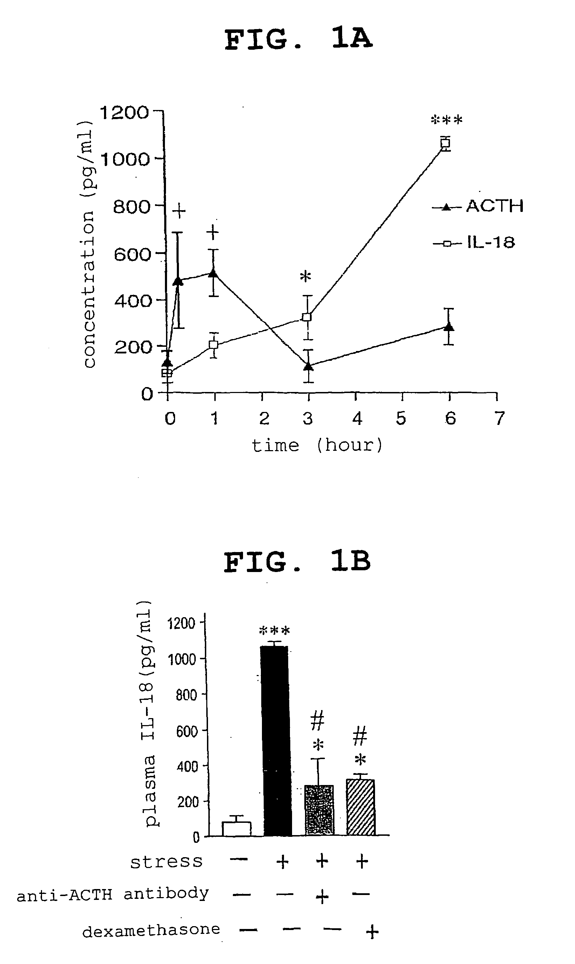

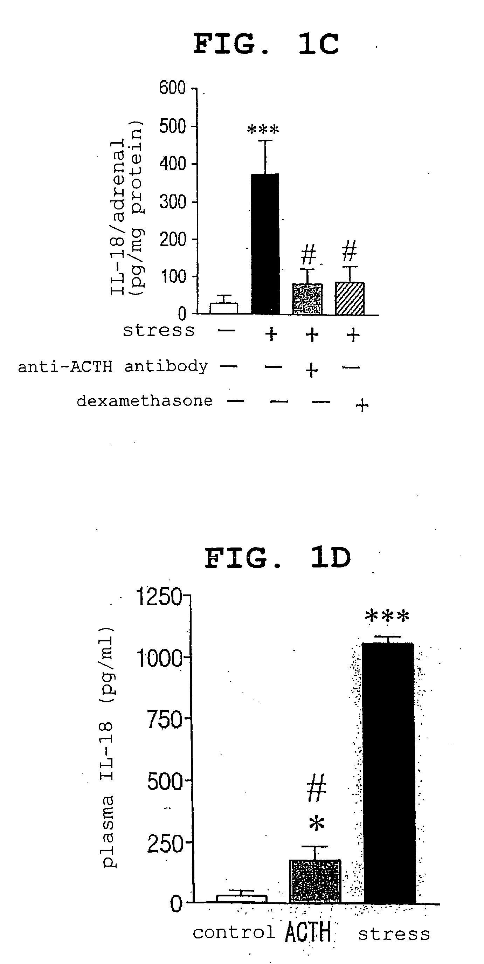

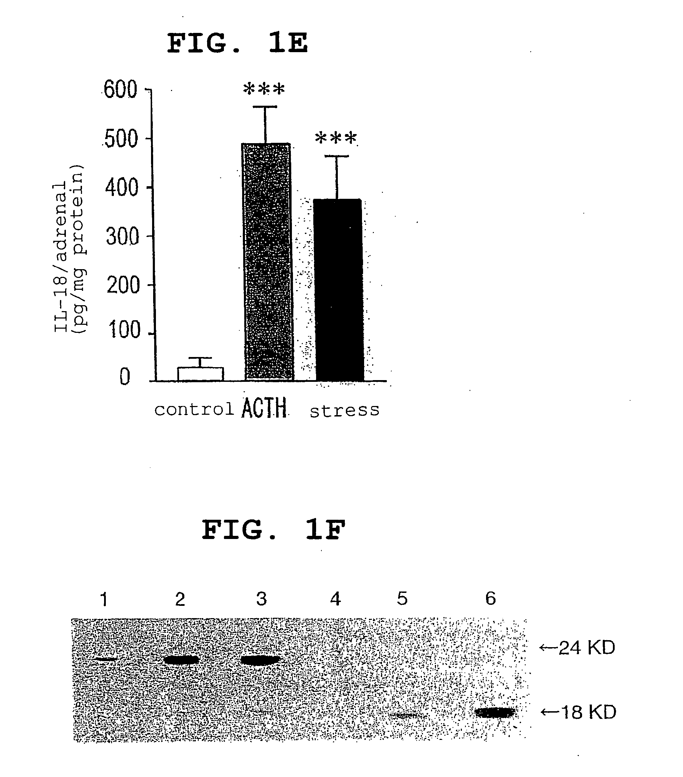

Giving restraint stress to an animal increases the level of the 24-kD IL-18 precursor protein in the adrenal, and the level of the 18-kD mature type in plasma (Fig. 1). These increases occur after adrenocorticotrophic hormone (ACTH) in plasma is elevated by the stress. This shows that in the adrenal, ACTH is involved in the induction of IL-18 precursor. This is supported by the discoveries that administration of anti-ACTH antiserum inhibits the increase in the levels of both mature type and precursor type IL-18, and that ACTH release induced by stress is inhibited by dexamethasone, whereby mature type and precursor type IL-18 are also inhibited. These results show that restraint stress induces ACTH, and then induces IL-18 precursor in the adrenal.

In the adrenal of a stress-loaded animal, not only IL-18 precursor protein but also mature type IL-18 is induced (Fig. 1F), suggesting that conversion from the precursor type to the mature type might occur due to the stress. Inhibiting the induction by ACTH of IL-18 precursor in the adrenal leads to the suppression of plasma IL-18 level. In summary, it is suggested that the IL-18 precursor in the adrenal induced by ACTH may be converted to the mature type and secreted in plasma. The conversion of the IL-18 precursor to the mature type is an important regulatory process in the accumulation of mature IL-18 in plasma in stress-loaded animals.

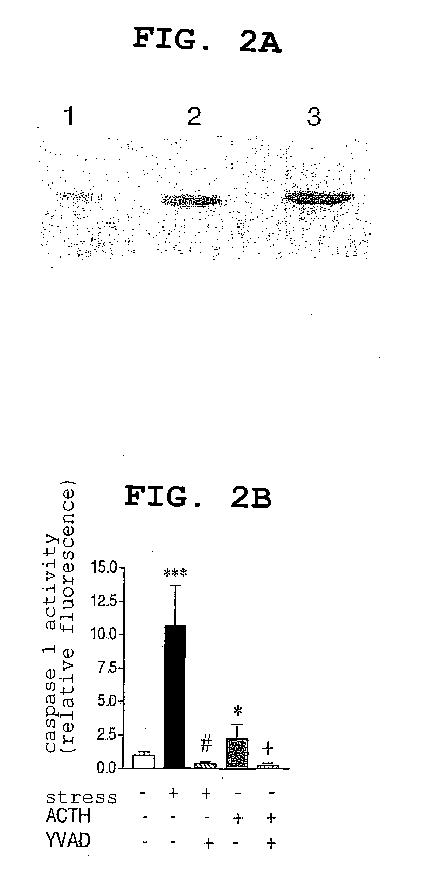

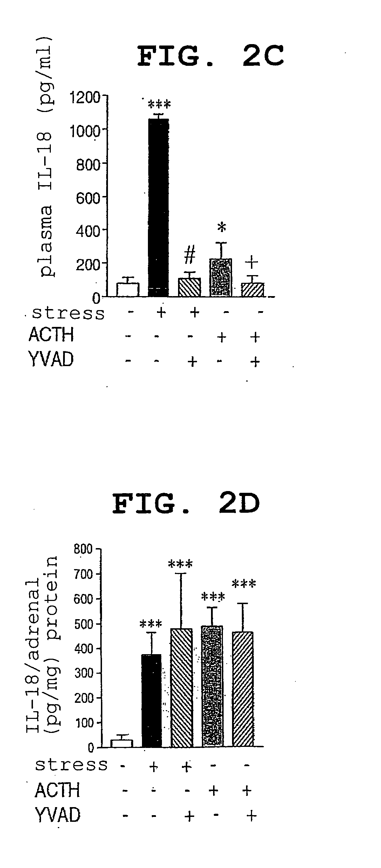

Caspase-1 precursor and the activity thereof are reported to be induced by LPS in the adrenal. In the present invention, it was shown that caspase-1 precursor was also induced by stress in the adrenal (Fig. 2A). YVAD-CHO, which is a caspase-1 inhibitor, inhibits the elevation of plasma IL-18 (Fig. 2B) but does not inhibit the induction of IL-18 precursor in the adrenal (Fig. 2D). In stress-loaded caspase-1 knockout (KO) animals, IL-18 precursor undergoes upregulation in the adrenal, but plasma IL-18 was undetectable. From these results, it is suggested that the caspase-1 induced by stress may have resulted in the processing of IL-18 precursor in the adrenal with respect to the accumulation of mature IL-18 in plasma.

Caspase-11 is reported to activate caspase-1. In the present invention, because Z-FA-fmk, which is an inhibitor of caspase-11, inhibited the activation of caspase-1 undergoing activation by stress, the involvement of caspase-11 in caspase-1 activation in stress-loaded animals is suggested. It has been reported that the induction of caspase-11 precursor requires the trans-activation of NF-κB, and that P38MAP kinase mediates the trans-activation of NF-κB. Furthermore, it has been reported that SB203580, which is a P38MAP kinase inhibitor, downregulates the induction of caspase-11.

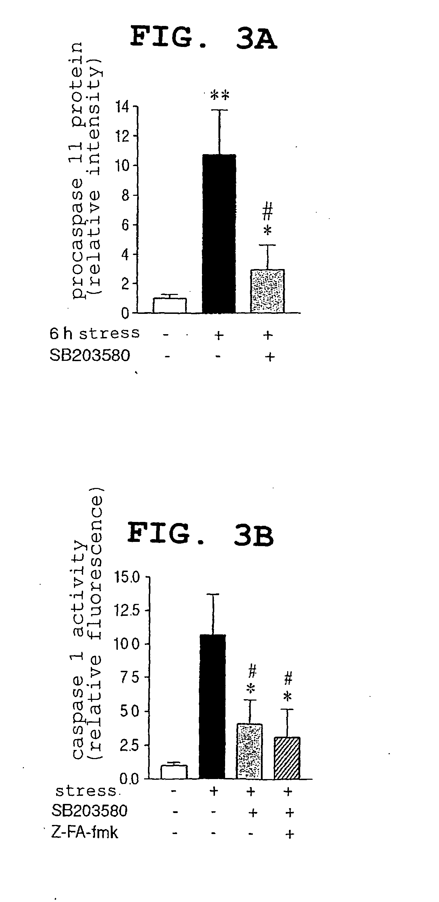

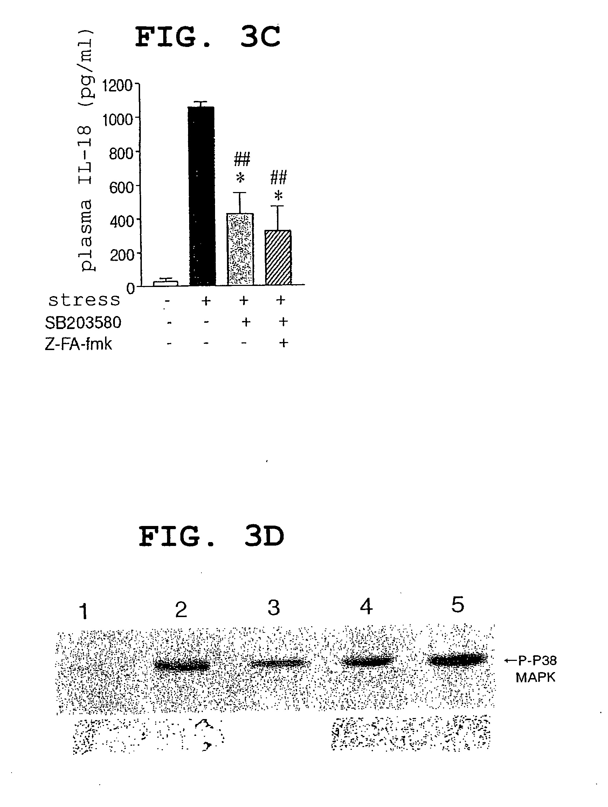

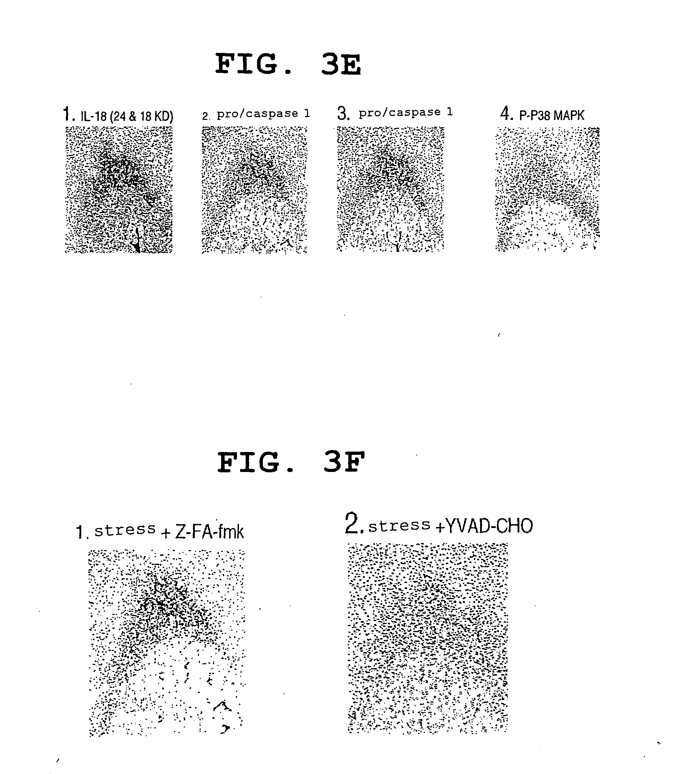

The present inventor discovered that the aforementioned SB203580 inhibited the stress-induced increases in the protein level of caspase-11 (Fig. 3A), caspase-1 activity (Fig. 3B) and plasma IL-18 level (Fig. 3C), respectively. The caspase-1 inhibitor YVAD-CHO and the caspase-11 inhibitor Z-FA-fmk suppress the stress-induced elevation of plasma IL-18 level without influencing the phosphorylation of P38MAP kinase (Figs. 3D and 3F). The stress-induced phosphorylation of P38MAP kinase protein occurred both in caspase-1 KO animals and in wild type animals (Fig. 3C). From these results, it is suggested that P38MAP kinase may be present upstream of caspase-11, form the P38MAP kinase → caspase-11 → caspase-1 cascade, and result in conversion from IL-18 precursor to the mature type in the adrenal, and release active type (mature type) IL-18 into plasma in stress-loaded animals.

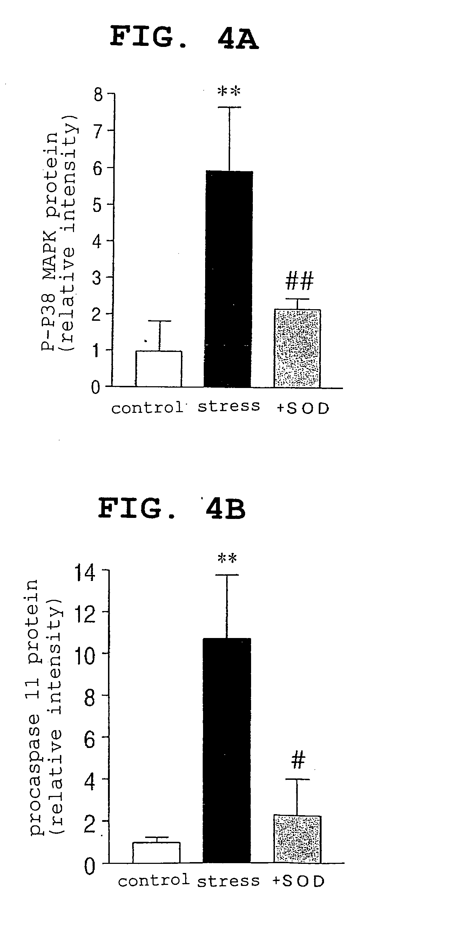

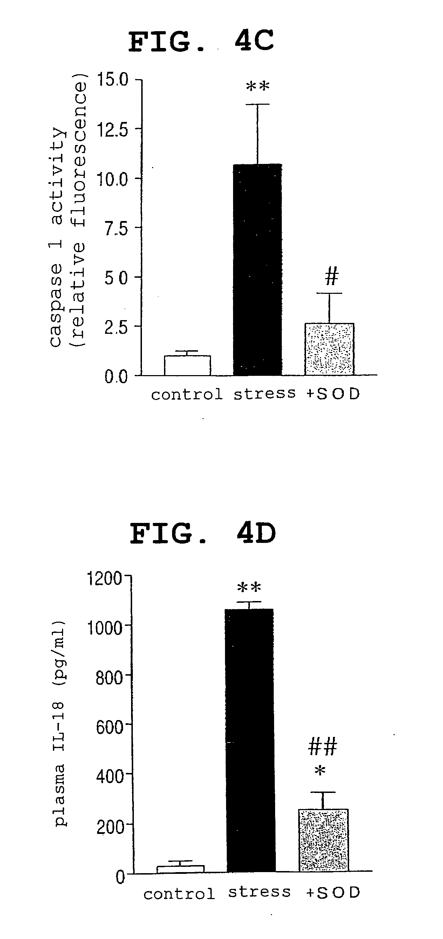

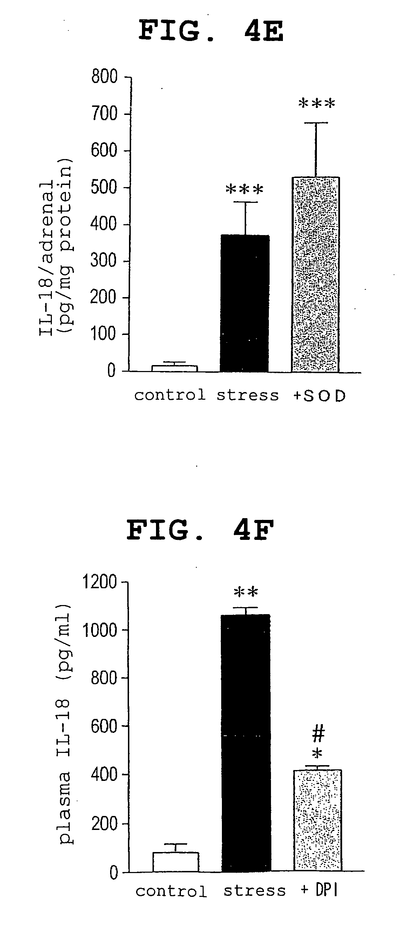

Active oxygen species are reported to mediate the phosphorylation of P38MAP kinase in pulmonary fibroblasts, neutrophils and monocytes. In the present invention, superoxide dismutase (SOD) inhibited the activation of P38MAP kinase (Fig. 4A), the induction of caspase-11, the activation of caspase-1 in the adrenal, and the elevation of plasma IL-18 level (Figs. 4B, C, D). However, the induction of IL-18 precursor in the adrenal was not influenced by SOD (Fig. 4E). From these results, it is suggested that stress may induce superoxide, which in turn may activate the P38MAP kinase cascade. Therefore, the process of generation of IL-18 in plasma in stress-loaded animals can be summarized as described below. Stress activates the hypothalamus-pituitary-adrenal (HPA) axis to induce ACTH. Also, stress generates superoxide, which in turn induces the phosphorylation of P38MAP kinase (Thr180/Tyr182), and the phosphorylated MAP kinase induces caspase-11, which caspase-11 then activates caspase-1. The thus-activated caspase-1 processes IL-18 precursor to form the mature type in the adrenal, and this mature type is secreted in plasma. This pathway is referred to as "the cytokine cascade in non-inflammatory stress (stress cascade)".

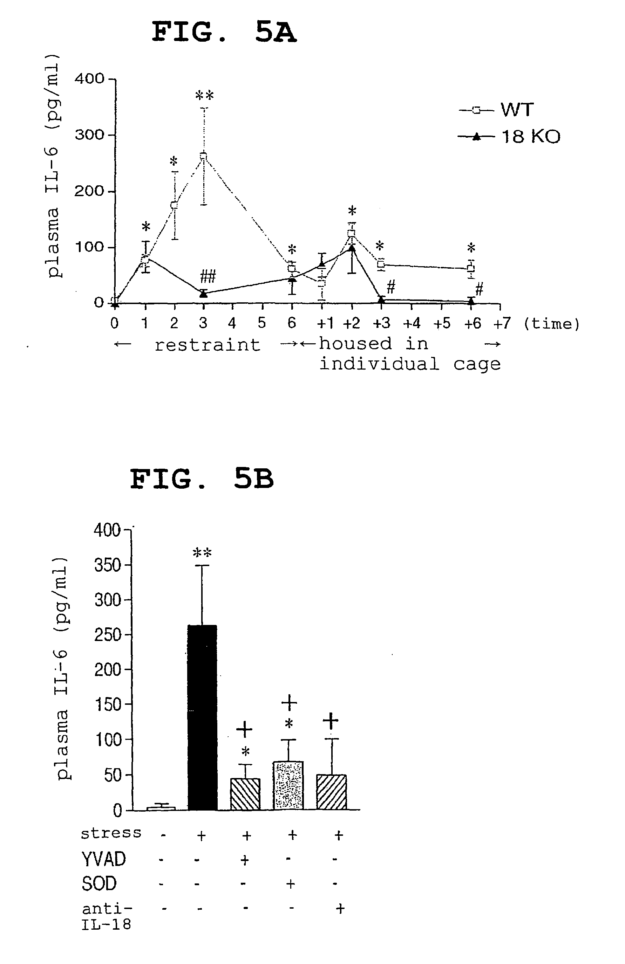

Because IL-18 activates TLR2 and Myd88, which are necessary for the induction of IL-6, the possibility that IL-18 induces IL-6 is suggested. The present inventor found that plasma IL-6 levels rose in stress-loaded wild type animals but did not rise in IL-18 KO animals (Fig. 5A). This suggests that IL-6 may be induced IL-18-dependently. This is supported by the result that when animals were treated with a caspase-1 inhibitor and SOD, the stress-induced elevations of IL-18 and IL-6 in plasma were inhibited, and the result that in stress-loaded animals, an anti-IL-18 antibody suppressed IL-6 levels. These results represent the first evidence that non-inflammatory stress raises plasma IL-6 levels IL-18-dependently.

Accordingly, the indicator agent of the present invention for non-inflammatory stress responses mediated by superoxide comprises an ingredient involved in the cytokine cascade in non-inflammatory stress. The ingredient involved in the cytokine cascade preferably comprises 1 or 2 or more proteins selected from the group consisting of IL-18, active type IL-18, P38MAP kinase, phosphorylated P38MAP kinase, caspase 11, caspase 1, and activated caspase 1, and more preferably comprises IL-18. Although the aforementioned IL-18 occurs as the 24-kD precursor or 18-kD mature form (or also referred to as the active type) protein in humans or mice, it is preferable, from the aforementioned finding, that the IL-18 be active type IL-18 present in blood, more preferably in plasma. The aforementioned active type IL-18 is exemplified by one having the 37th to 193rd amino acids in the amino acid sequence of SEQ ID NO:2 in the case of humans.

Judging from the aforementioned finding, the indicator agent of the present invention preferably comprises P38MAP kinase in addition to IL-18. The aforementioned P38MAP kinase is more preferably a phosphorylated one, and it is more preferable that the phosphorylation site of the aforementioned P38MAP kinase be Thr180 or Tyr182 in the case of mice, or Thr185 or Tyr187 in the case of humans.

Judging from the aforementioned finding, the indicator agent of the present invention preferably comprises caspase-11 in addition to IL-18 and P38MAP kinase.

Judging from the aforementioned finding, the indicator agent of the present invention preferably comprises caspase-1 in addition to IL-18, P38MAP kinase and caspase-11. Although the aforementioned caspase-1 may be procaspase-1 or activated caspase-1 (mature form), activated caspase-1 is preferable because it results in the processing of IL-18 precursor. The aforementioned activated caspase-1 is exemplified by one having the 120th to 404th amino acids in the amino acid sequence of SEQ ID NO:8 in the case of humans.

Although the indicator agent of the present invention may comprise 1 kind or 2 kinds or more of P38MAP kinase, caspase-11 and caspase-1, as long as it comprises at least IL-18, the aforementioned cytokines and enzymes that constitute the indicator agent are preferably housed in separate containers. Also, the indicator agent of the present invention may comprise another ingredient, as long as it has any of the aforementioned cytokines and enzymes as the major ingredient. As the other ingredient, solvents such as buffer solutions and physiological saline, stabilizers, bacteriostatic agents, antiseptics and the like can be mentioned, but are not to be construed as limiting.

The indicator agent of the present invention serves as an index for the exposure and responding of an animal to non-inflammatory stress, preferably physical stress, chemical stress, mental stress and oxidative stress, more preferably stress due to ultraviolet irradiation, stress due to chemical substance exposure, restraint stress and oxidative stress caused thereby, particularly preferably to restraint stress. Particularly, by using active type IL-18 in blood, preferably in plasma, of an animal, as the index, the degree of stress can be known easily and quantitatively. The amount of IL-18 in plasma, in the case of mice, for example, is not more than 100 pg/ml before stress loading, becomes 120 to 200 pg/ml at 5 hours after 1 hour of stress loading, and exceeds 1000 pg/ml after 6 hours of stress loading; as stress loading increases, the amount of IL-18 increases. Furthermore, also because the half-life of IL-18 is about 10 hours, an indicator agent comprising IL-18 is suitable as an index for non-inflammatory stress responses. In addition to IL-18, an elevation of the phosphorylation of P38MAP kinase in the adrenal, an increase in the amount of caspase-11, or an elevation of the activity of caspase-1 also serves as an index for non-inflammatory stress responses. Specifically, it is preferable to supply a range of concentrations of IL-18 contained in the indicator agent of the present invention, from a normal value to concentrations causing weak stress to strong stress, in preparation for a comparison with samples.

Also, because the indicator agent of the present invention contains ingredients involved in the stress cascade, including IL-18, it can be utilized for screening of a substance that reduces these ingredients and activities. Although the screening method using the indicator agent of the present invention is not subject to limitation; examples include a method comprising administering various substances to a stress-loaded animal, and selecting a substance that significantly reduces the amount of active type IL-18 in blood. Substances selected by such a screening method can be used as active ingredients for a pharmaceutical or a food with health claims, which enable the management of stress.

The visualizing agent of the present invention for non-inflammatory stress responses is intended to detect the aforementioned indicator agent. Therefore, the visualizing agent of the present invention is not subject to limitation, as long as it detects at least 1 kind or 2 kinds or more of IL-18, P38MAP kinase, caspase-11 and caspase-1; the agent comprising various compounds that bind specifically to the aforementioned cytokines or enzymes, and one comprising a specific substrate for the aforementioned enzymes can be mentioned.

The visualizing agent of the present invention preferably comprises an IL-18 antibody for detecting IL-18, and the aforementioned IL-18 antibody is more preferably one that specifically recognizes active type IL-18.

The visualizing agent of the present invention preferably further comprises a P38MAP kinase antibody in addition to the IL-18 antibody, and the P38MAP kinase antibody is preferably an anti-phosphorylated P38MAP kinase antibody. The aforementioned anti-phosphorylated P38MAP kinase antibody more preferably recognizes a P38MAP kinase having phosphorylated Thr180 or Tyr182 in the case of mice, and recognizes a P38MAP kinase having phosphorylated Thr185 or Tyr187 in the case of humans.

The visualizing agent of the present invention preferably further comprises a caspase-11 antibody in addition to the IL-18 antibody and P38MAP kinase antibody.

The visualizing agent of the present invention preferably further comprises caspase-1 antibody, in addition to the IL-18 antibody, P38MAP kinase antibody and caspase-11. The aforementioned caspase-1 antibody is more preferably one that specifically recognizes activated caspase-1.

The visualizing agent of the present invention preferably further comprises a substrate enabling a measurement of caspase-1 activity, in addition to the IL-18 antibody, P38MAP kinase antibody and caspase-11. The aforementioned substrate is more preferably one that emits fluorescence upon cleavage with enzyme, in order to facilitate its detection. As examples of such a substrate, Ac-YVAD-MCA, which is described in an Example, can be mentioned.

The visualizing agent of the present invention preferably comprises at least an IL-18 antibody, and may further comprise 1 kind or 2 kinds or more of P38MAP kinase antibody, caspase-11 antibody, caspase-1 antibody and caspase-1 substrate, but the aforementioned antibody or substrate that constitutes the visualizing agent is preferably housed in separate containers. Also, the visualizing agent of the present invention may comprise another ingredient, as long as it has the aforementioned antibody or substrate as the major ingredient. As the other ingredient, buffer solutions, solvents such as physiological saline, stabilizers, bacteriostatic agents, antiseptics and the like can be mentioned, but are not to be construed as limitative.

The aforementioned antibody or substrate is useful as a tool (stress marker) enabling a determination of whether or not a subject is exposed to non-inflammatory stress or a measurement of the degree of the stress response thereof by detecting the presence or absence or degree of the aforementioned indicator agent in the subject.

Also, the aforementioned antibody is also useful as a tool (stress marker) for detecting a change in the expression of any of IL-18, P38MAP kinase, caspase-11 and caspase-1 in the prevention, amelioration or prediction of a change in immune status based on non-inflammatory stress described below.

The aforementioned antibody is not subject to limitation as to the form thereof, and may be a polyclonal antibody against any of IL-18, P38MAP kinase, caspase-11 and caspase-1 as the immune antigen, or a monoclonal antibody thereof. Furthermore, antibodies having antigen bindability to polypeptides consisting of usually at least 8 consecutive amino acids, preferably 15 amino acids, more preferably 20 amino acids, in the amino acid sequences of these proteins, are also included in the antibody of the present invention.

Methods of producing these antibodies are publicly known, and the aforementioned antibody can also be produced according to a conventional method (Current Protocol in Molecular Biology, Chapter 11.12 to 11.13 (2000)). Specifically, when the antibody of the present invention is a polyclonal antibody, the antibody can be obtained by using IL-18, P38MAP kinase, caspase-11 or caspase-1 expressed and purified in Escherichia coli and the like according to a conventional method, or by synthesizing an oligopeptide having a partial amino acid sequence of these proteins according to a conventional method, and immunizing a non-human animal such as a domestic rabbit therewith, and separating the antibody from the serum of the immunized animal according to a conventional method. Meanwhile, in the case of a monoclonal antibody, the antibody can be obtained hybridoma cells prepared by immunizing a non-human animal such as a mouse with IL-18, P38MAP kinase, caspase-11 or caspase-1 expressed in Escherichia coli and purified and the like according to a conventional method, or an oligopeptide having a partial amino acid sequence of these proteins, and cell-fusing the splenocytes obtained and myeloma cells (Current protocols in Molecular Biology edit. Ausubel et al. (1987) Publish. John Wiley and Sons. Section 11.4 to 11.11).

The IL-18, P38MAP kinase, caspase-11 or caspase-1 used as the immune antigen in preparing an antibody, and included in the indicator agent of the present invention, can be obtained on the basis of the sequence information on the genes provided by the present invention (SEQ ID NO:1, 3, 5, 7), by operations of DNA cloning, construction of each plasmid, transfection to a host, cultivation of a transformant, and protein recovery from the culture. These operations can be performed by a method known to those skilled in the art, or in accordance with methods described in the literature (Molecular Cloning, T. Maniatis et al., CSH Laboratory (1983), DNA Cloning, DM. Glover, IRL PRESS (1985)) and the like.

Specifically, by preparing a recombinant DNA (expression vector) that allows the gene encoding IL-18, P38MAP kinase, caspase-11 or caspase-1 to be expressed in a desired host cell, introducing the same into a host cell to transform the cell, culturing the transformant, and recovering the desired protein from the culture obtained, a protein as an immune antigen for producing the antibody of the present invention can be obtained. Also, partial peptides of these IL-18, P38MAP kinase, caspase-11 or caspase-1 can also be produced by a common chemical synthesis method (peptide synthesis) according to the amino acid sequence information provided by the present invention (SEQ ID NO:2, 4, 6, 8). For producing an anti-phosphorylated P38MAP kinase antibody, a peptide containing phosphorylated Thr or Tyr may be synthesized.

The method of the present invention of measuring the degree of non-inflammatory stress comprises measuring the amount or activity of the indicator agent of the present invention. As the indicator agent, IL-18 is preferable, and active type IL-18 is more preferable.

In the above-described method, the "non-inflammatory stress" is preferably physical stress, chemical stress, mental stress or oxidative stress, more preferably stress due to ultraviolet irradiation, stress due to chemical substance exposure, restraint stress, or oxidative stress caused thereby, and particularly preferably restraint stress.

As the subject of measurement by the present method, blood, body fluid, tissue, cells, and excretes collected from an animal or extracts thereof, and cell extract (extract) obtained from cultured cells, cell culture broth, and the like can be mentioned, and blood collected from an animal and a culture broth of cultured cells are preferable. As examples of the aforementioned animal, human, bovine, horse, swine, sheep, goat, dog, cat, mice, rats, rabbit, goat, hamster, chicken and the like can be mentioned. As the aforementioned cultured cells, cells having the capability of producing IL-18 are preferable; for example, synovial cells, renal tubular epithelial cells, microglia, astrocytes, macrophages, keratinocytes and the like can be mentioned.

In the above-described method, the detection, quantitation and the like of the indicator agent can be performed by any conventionally known method, but it is preferable to use the visualizing agent of the present invention; the detection, quantitation and the like of the indicator agent can be performed using these visualizing agents according to, for example, ELISA, Western blot analysis and the like and methods based thereon. Because the aforementioned visualizing agent has the property to bind specifically to IL-18, P38MAP kinase, caspase-11 or caspase-1, the aforementioned cytokines or enzymes expressed in animal tissue can be detected specifically.

The above-described method, for example, when IL-18 is used as the index, enables an easy measurement because no more than the detection of active type IL-18 released into blood or plasma is necessary. As described above, the amount of IL-18 in plasma, in the case of mice, for example, is not more than 100 pg/ml before stress loading, becomes 120 to 200 pg/ml at 5 hours after 1 hour of stress loading, and exceeds 1000 pg/ml after 6 hours of stress loading; as stress loading increases, the amount of IL-18 also increases. Furthermore, also because the half-life of IL-18 is about 10 hours, an indicator agent comprising IL-18 is suitable as an index for non-inflammatory stress responses. Using cultured cells in place of an animal, IL-18 released in the medium (or culture broth) may be detected and quantified.

Also, in the case of caspase-11 or caspase-1, the degree of stress can also be measured by detecting the product resulting from the reaction with the aforementioned substrate. Specifically, for example, a substrate for caspase-11 or caspase-1 manipulated to produce fluorescence upon cleavage is administered to an animal, and fluorescence released into blood upon cleavage of the substrate in the adrenal can be detected. When cultured cells are used in place of an animal, fluorescence in cell extract can be detected and quantified. From the intensity of fluorescence detected, the degree of elevation of the aforementioned enzyme activity is known, and by comparing the elevation with a predetermined reference value, the degree of non-inflammatory stress can be measured. Alternatively, by administering to an animal the above-described antibody conjugated with a fluorescent agent, a radioactive molecule or a metal, and examining the accumulation of the fluorescence, radioactive molecule or metal in the adrenal using CT (computed tomography); MRI (magnetic resonance imaging), PET (positron emission tomography) or SPECT (single photon emission computed tomography) and the like, IL-18, P38MAP kinase, caspase-11 or caspase-1 can be detected. From the detected amount accumulated, the presence or absence or abundance of each ingredient of these stress response indicator agents is known, and by making a comparison with a predetermined reference value, the degree of stress in the sample can be measured.

According to the present invention, an assay kit for non-inflammatory stress comprising the above-described visualizing agent is provided.

The visualizing agent contained in the kit of the present invention is not subject to limitation, as long as it detects at least 1 kind or 2 kinds or more of IL-18, P38MAP kinase, caspase-11 and caspase-1, and the agent comprising various compounds that bind specifically to the aforementioned cytokines or enzymes, and one comprising a specific substrate for the aforementioned enzymes can be mentioned, but it is preferable that an IL-18 antibody be contained, and the aforementioned IL-18 antibody is more preferably one that specifically recognizes active type IL-18. According to the kit of the present invention, it is possible to measure the degree of non-inflammatory stress in the subject of measurement conveniently and quickly. Also, in addition to the visualizing agent of the present invention, the assay kit of the present invention can further comprise other optionally chosen reagents necessary in performing a measurement and detection. Specifically, for example, fluorescently labeled IL-18, active type IL-18, assay buffer solution and the like can be mentioned as examples.

In the above-described kit, the "non-inflammatory stress" is preferably physical stress, chemical stress, mental stress or oxidative stress, more preferably stress due to ultraviolet irradiation, stress due to chemical substance exposure, restraint stress, or oxidative stress caused thereby, and particularly preferably restraint stress.

In diagnosing a change in immune status based on a non-inflammatory stress response, it is preferable to predetermine a normal value (reference value) of IL-18 in blood according to the kind of animal, to measure the amount of IL-18 in blood of the subject of diagnosis, and to compare the measured value with the normal value. If the measured value is significantly higher than the normal value (reference value), it can be judged that a change in immune status has occurred.

In the present invention, because a non-inflammatory stress response mediated by superoxide has caused a change in immune status, it is preferable to further measure a peroxide in blood, spinal fluid, urine or saliva produced by superoxide in the aforementioned assay method or assay kit, or in the prophylactic, ameliorating or predicting method described below.

The aforementioned peroxide is not subject to limitation; peroxidized lipids produced upon peroxidation reactions of lipids, such as acrolein, 4-hydroxynonenal, malondialdehyde, 4-hydroxy-2-hexenal, and crotonaldehyde, peroxidated saccharides such as methylglyoxal, and oxidized bases such as 8-hydroxy-2'-deoxyguanosine can be mentioned. Confirmation of a significant elevation of peroxide and a significant elevation of the indicator agent according to the present invention is preferable because it provides a strong piece of evidence that the animal has an change in immune status.

Of the aforementioned visualizing agents, a visualizing agent comprising an antibody enables the prevention, amelioration or prediction of a change in immune status based on a non-inflammatory stress response when applied to an animal, and the present invention provides such a method.

The aforementioned animal is preferably a vertebral animal other than a human, and is particularly preferably a livestock or companion animal such as a bovine, horse, swine, sheep, goat, chicken, dog, or cat. By applying the prophylactic, ameliorating or predicting method of the present invention to a livestock or companion animal, an effect of keeping the animal in a good immune status and rearing animals unlikely to contract disease can be obtained in the field of animal husbandry or pet industry, in which artificial rearing is likely to lead to stress loading.

The present invention provides a therapeutic agent for a change in immune status based on a non-inflammatory stress response for reducing the amount or activity of the aforementioned indicator agent.

The therapeutic agent of the present invention is intended to reduce the amount or activity of the aforementioned indicator agent. Therefore, the therapeutic agent of the present invention is not subject to limitation, as long as it reduces the amount or activity of at least 1 kind or 2 kinds or more of IL-18, P38MAP kinase, caspase-11 and caspase-1, and those containing various compounds that bind specifically to the aforementioned cytokines or enzymes or a suppressant of superoxide (also referred to as antioxidant substance or antioxidant agent) as the active ingredient can be mentioned.

As the aforementioned compound, the aforementioned various antibodies can be mentioned, and the compound is preferably an IL-18 antibody or an anti-IL-18 receptor antibody, more preferably an antibody that binds specifically to active type IL-18.

As the aforementioned suppressant of superoxide, thiol compounds, antioxidant peptides/amino acids, food ingredients such as various plant-derived polyphenols, soybean lecithin, cholesterol, chlorophyll, herb extracts (for example, Carnosol), wasabi extract, sulforaphane and derivative analogues thereof, isothiocyanate and derivative analogues thereof, 6-(meththylsufinyl)hexyl isothiocyanate, carotenoid, vitamin B2, vitamin E, zinc, glutathion, glutathion peroxidase, catalase, curcumin, sesame lignin, vitamin C (including ascorbate derivatives), carotenes (for example, β-carotene, lycopene), xanthophylls (for example, astaxanthin), eicosapentaenoic acid (EPA), coenzyme Q₁₀, pycnogenol, oleic acid, and linoleic acid, enzyme-derived ingredients such as superoxide dismutase (SOD), diphenylene iodonium chloride (DPI), and myeloperoxidase/NADPH oxidase antibodies, hormones such as insulin, estrogen, melatonin, and IGF (insulin-like growth factor), brain protecting agents (for example, edaravone (trade name)), NO (nitrogen oxide) donor compounds such as DETA NONOate (diethylenetriamine NONOate) and the like can be mentioned. Also, as secondary suppressants, phospholipase suppressants such as quinacrine, arachidonic acid liberation suppressants such as indomethacin, therapeutic drugs for hyperlipemia such as probucol, oral hypoglycemic agents such as AT1 blockers and ACE inhibitors, and tranquilizers such as minor tranquilizers can be mentioned, and the suppressant is preferably SOD, vitamin E, or coenzyme Q₁₀, more preferably SOD or vitamin E.

The therapeutic agent of the present invention may be the aforementioned active ingredient as is, and may be a pharmaceutical composition prepared by mixing with a known pharmaceutically acceptable carrier (excipient, filler, binder, lubricant and the like are included), commonly used additives and the like. The pharmaceutical composition may be prepared in various forms according to the purpose thereof, for example, oral formulations such as tablets, pills, capsules, powders, granules, and syrups; non-oral formulations such as injections, drip infusions, external preparations, and suppositories, and the like.

As examples of the above-described carrier, excipients such as lactose, glucose, starch, calcium carbonate, kaolin, crystalline cellulose, and silicic acid, binders such as water, ethanol, propyl alcohol, simple syrup, glucose solution, starch solution, gelatin solution, carboxymethylcellulose, shellac, methylcellulose, and polyvinylpyrrolidone, disintegrants such as dry starch, sodium alginate, agar powder, carmellose calcium, starch, and lactose, disintegration suppressants such as sucrose, cacao butter, and hydrogenated oil, absorption promoters such as quarternary ammonium bases and sodium lauryl sulfate, moisture retainers such as glycerine and starch, adsorbents such as starch, lactose, kaolin, bentonite, and colloidal silica, lubricants such as talc, stearates, and polyethylene glycol, and the like can be used.

Furthermore, tablets can be prepared as tablets having an ordinary coating as required, for example, sugar-coated tablets, gelatin-coated tablets, enteric coated tablets, film-coated tablets or double-layer tablets or multiple-layer tablets.

When the therapeutic agent of the present invention is molded into the form of pills, carriers conventionally known in the relevant field can be used widely. As examples thereof, excipients such as crystalline cellulose, lactose, starch, hardened vegetable oil, kaolin, and talc, binders such as acacia powder, tragacanth powder, and gelatin, disintegrants such as carmellose calcium and agar, and the like can be mentioned.

Capsules are prepared by mixing an ordinary active ingredient compound with various carriers exemplified above according to a conventional method, and filling the mixture in hard gelatin capsules, soft capsules and the like.

When prepared as injections, solutions, emulsions and suspensions are preferably sterilized and isotonic to blood; in molding into these forms, diluents in common use in the relevant field, for example, water, ethanol, macrogol, propylene glycol, ethoxidated isostearyl alcohol, polyoxydated isostearyl alcohol, polyoxyethylene sorbitan fatty acid esters and the like, can be used. In this case, sodium chloride, glucose or glycerine in an amount necessary to prepare an isotonic solution may be contained in the pharmaceutical preparation, and an ordinary solubilizer, buffering agent, soothing agent and the like may be added.

In molding into the form of suppositories, conventionally known carriers can be used widely. As examples thereof, semisynthetic glycerides, cacao fat, higher alcohols, esters of higher alcohols, polyethylene glycol and the like can be mentioned.

Furthermore, as required, a colorant, a preservative, a flavoring agent, a corrective, a sweetening agent and the like, and another pharmaceutical, can be contained in the pharmaceutical preparation. Although the amount of active ingredient compound to be contained in these pharmaceutical preparations of the present invention is chosen as appropriate from a broad range without limitation, it is usually recommended that the amount be about 1 to 70% by weight, preferably about 5 to 50% by weight, in the preparation composition.

The method of administration of these pharmaceutical preparations of the present invention is not subject to limitation, and the preparations can be administered orally or non-orally by a method according to various preparation forms, patient age, sex, severity of disease and other conditions. For example, in the case of tablets, pills, solutions, suspensions, emulsions, granules and capsules, the preparations are administered orally; in the case of injections, the preparations are administered intravenously, alone or in a mixture with an ordinary fluid replacement such as glucose and amino acids, and, as required, administered alone intramuscularly, subcutaneously or intraperitoneally. In the case of suppositories, the preparations are administered intrarectally.

Also, the dose varies depending on the kind of active ingredient, route of administration, age, body weight and symptoms of recipient or patient, and the like, and cannot be generally determined; usually, about several milligrams to 2 g, preferably about several tens of milligrams, as the daily dose, can be administered in one to several divided portions in a day.

The disease (disease that occurs, exacerbates, or recurs due to non-inflammatory stress) or condition to be treated is not subject to limitation, as long as it is based on an immunological change resulting from a non-inflammatory stress response mediated by superoxide, and this includes diseases that occur, exacerbate or recur with the involvement of IL-18 and IL-18-induced cytokines. Specifically, autoimmune diseases, exacerbation or recurrence of autoimmune diseases, mental diseases, malignant tumors and the like can be mentioned.

Specifically, as the diseases that occur, exacerbate or recur due to non-inflammatory stress, appendicitis, digestive ulcers, gastric ulcer, duodenal ulcer, peritonitis, pancreatitis, ulcerative, pseudomembranous, acute and ischemic colitis, diverticulitis, epiglottitis, achalasia, cholangitis, cholecystitis, hepatitis, Crohn's disease, enterocolitis, Whipple's disease, (bronchial) asthma, allergies, anaphylactic shock, immune complex disease, ischemia of organs, reperfusion disorders, necrosis of organs, hay fever, sepsis, septicemia, endotoxin shock, cachexia, hyperpyrexia, eosinophilic granuloma, granulomatous disease, sarcoidosis, septic abortion, epididymitis, vaginitis, prostatitis, urethritis, bronchitis, pulmonary emphysema, pulmonary edema, rhinitis, cystic fibrosis, pneumonia, pneumoconiosis, alveolitis, bronchiolitis, pharyngitis, pleurisy, sinusitis, influenza, respiratory syncytial virus infection, herpes infection, HIV infection, hepatitis B virus infection, hepatitis C virus infection, disseminated bacteremia, dengue fever, candidiasis, malaria, filariasis, amebiasis, hydatid cysts, burns, dermatitis, dermatomyositis, sunburns, urticaria, warts, wheals, angiitis, vasculitis, endocarditis, arteritis, atherosclerosis, thrombophlebitis, pericarditis, myocarditis, myocardial ischemia, periarteritis nodosa, rheumatic fever, Alzheimer's disease, Alzheimer type senile dementia, diffuse Lewy body disease, Pick's disease, Binswanger's disease, Parkinson's disease, Parkinson syndrome, cerebral ischemia, cerebral ischemia/reperfusion disorders, neonatal hemorrhagic encephalopathy, epilepsy, HIV encephalopathy, brain circulatory disorders, cerebrovascular disorders, celiac disease, congestive heart failure, adult respiration distress syndrome, meningitis, encephalitis, multiple sclerosis, cerebral infarction, cerebral stroke, Guillain-Barré syndrome, neuritis, neuralgia, spinal injury, paralysis, uveitis, arthritic eruption, arthralgia, osteomyelitis, fasciitis, Paget's disease, articular rheumatism, arthritis, gout, periodontal disease, rheumatoid arthritis, synovitis, myasthenia gravis, thyroiditis, systemic lupus erythematosus (SLE), Goodpasture's syndrome, Behçet's syndrome, graft rejection in homologous transplantation, graft-versus-host disease, type I diabetes mellitus, ankylosing spondylitis, Buergur's disease, type II diabetes mellitus, ankylosing spondylitis, Buergur's disease, Reiter's syndrome, Hodgkin's disease, liver cirrhosis, renal insufficiency, allergic asthma, nephritis, myocardial infarction, Sjoegren's syndrome, contact dermatitis, atopic dermatitis, depression, eating disorders, allotriophagy, taste disorders, acne vulgaris, pemphigus, ichthyosis, psoriasis, burns, photosensitivity, ultraviolet skin disorders, wound healing, or radiation disorders, carbon monoxide poisoning, thinner poisoning, hypoxia or secondary disorders thereto, endometriosis, adrenal insufficiencies, emmeniopathy, cardiomyopathy, psychosomatic disorders, amyotrophic lateral sclerosis (ALS), oligospermia, sexual desire reduction, hypopituitarism, pituitary insufficiencies and the like can be mentioned.

As the aforementioned autoimmune disease or mental disease, appendicitis, digestive ulcers, gastric ulcer, duodenal ulcer, peritonitis, pancreatitis, ulcerative, pseudomembranous, acute and ischemic colitis, diverticulitis, epiglottitis, achalasia, cholangitis, cholecystitis, hepatitis, Crohn's disease, enterocolitis, Whipple's disease, asthma, allergies, anaphylactic shock, immune complex disease, ischemia of organs, reperfusion disorders, necrosis of organs, hay fever, sepsis, septicemia, endotoxin shock, cachexia, hyperpyrexia, eosinophilic granuloma, granulomatous disease, sarcoidosis, septic abortion, epididymitis, vaginitis, prostatitis, urethritis, bronchitis, pulmonary emphysema, rhinitis, cystic fibrosis, pneumonia, pneumoconiosis, alveolitis, bronchiolitis, pharyngitis, pleurisy, sinusitis, influenza, respiratory syncytial virus infection, herpes infection, HIV infection, hepatitis B virus infection, hepatitis C virus infection, disseminated bacteremia, dengue fever, candidiasis, malaria, filariasis, amebiasis, hydatid cysts, burns, dermatitis, dermatomyositis, sunburns, urticaria, warts, wheals, angiitis, vasculitis, endocarditis, arteritis, atherosclerosis, thrombophlebitis, pericarditis, myocarditis, myocardial ischemia, periarteritis nodosa, rheumatic fever, Alzheimer's disease, celiac disease, congestive heart failure, adult respiration distress syndrome, meningitis, encephalitis, multiple sclerosis, cerebral infarction, cerebral stroke, Guillain-Barré syndrome, neuritis, neuralgia, spinal injury, paralysis, uveitis, arthritic eruption, arthralgia, osteomyelitis, fasciitis, Paget's disease, gout, periodontal disease, rheumatoid arthritis, synovitis, myasthenia gravis, thyroiditis, systemic lupus erythematosus, Goodpasture's syndrome, Behçet's syndrome, graft rejection in homologous transplantation, graft-versus-host disease, type I diabetes mellitus, ankylosing spondylitis, Buergur's disease, type II diabetes mellitus, ankylosing spondylitis, Buergur's disease, Reiter's syndrome, Hodgkin's disease, SLE, liver cirrhosis, renal insufficiency, allergic asthma, nephritis, myocardial infarction, Sjoegren's syndrome, contact dermatitis, atopic dermatitis, depression, eating disorders, allotriophagy, taste disorders, acne vulgaris, pemphigus, ichthyosis, psoriasis, burns, photosensitivity, ultraviolet skin disorders, or radiation disorders, carbon monoxide poisoning, hypoxia or secondary disorders thereto and the like can be mentioned.

As the malignant tumors, hematopoietic organ tumors such as acute leukemia, chronic leukemia, malignant lymphoma, multiple myeloma, and macroglobulinemia, as well as solid tumors such as colorectal cancer, brain tumors, head-and-neck cancer, breast cancer, lung cancer, esophageal cancer, gastric cancer, liver cancer, gall bladder cancer, cholangiocarcinoma, pancreas cancer, islet cell carcinoma, renal cell carcinoma, adrenal cortex cancer, urinary bladder cancer, prostatic cancer, testicular tumor, ovarian cancer, uterine cancer, choriocarcinoma, thyroid cancer, malignant cartinoid tumor, skin cancer, malignant melanoma, osteosarcoma, soft tissue sarcoma, neuroblastoma, Wilms tumor, and retinoblastoma, can be mentioned.

An elevation of IL-18 in blood is followed by the release of various cytokines. In particular, IL-6 is involved in many inflammatory diseases and is drawing attention as an exacerbation factor. As diseases known to be caused or exacerbated due to a systemic elevation of these cytokines, out of the above-described diseases, diseases involved by inflammatory mechanisms, such as appendicitis, peritonitis, pancreatitis, ulcerative, pseudomembranous, acute and ischemic colitis, diverticulitis, epiglottitis, cholangitis, cholecystitis, hepatitis, epididymitis, vaginitis, prostatitis, urethritis, bronchitis, pneumonia, pneumoconiosis, alveolitis, bronchiolitis, pharyngitis, pleurisy, sinusitis, dermatitis, dermatomyositis, angiitis, vasculitis, endocarditis, arteritis, atherosclerosis, thrombophlebitis, pericarditis, myocarditis, myocardial ischemia, periarteritis nodosa, osteomyelitis, fasciitis, rheumatic fever, meningitis, encephalitis, Paget's disease, gout, periodontal disease, thyroiditis, neuritis, uveitis, type I diabetes mellitus, gastric ulcer, digestive ulcers, and duodenal ulcer; diseases known to be involved by cytokines or autoimmune mechanisms, such as Crohn's disease, ulcerative colitis, Sjoegren's syndrome, multiple sclerosis, Guillain-Barré syndrome, dermatomyositis, systemic lupus erythematosus, bronchial asthma, immune complex disease, eosinophilic granuloma, granulomatous disease, sarcoidosis, articular rheumatism, periarteritis nodosa, ankylosing spondylitis, Behçet's syndrome, graft rejection in homologous transplantation, graft-versus-host disease, malignant hyperpyrexia, habitual abortion, and endometriosis; infectious diseases such as hay fever, influenza, respiratory syncytial virus infection, herpes infection, HIV infection, hepatitis B virus infection, hepatitis C virus infection, disseminated bacteremia, dengue fever, candidiasis, malaria, filariasis, amebiasis, and hydatid cysts; and diseases that have not completely been clarified pathologically but are shown to be involved by the above-described cytokines, such as achalasia, Whipple's disease, burns, sunburns, urticaria, warts, wheals, angiitis, vasculitis, endocarditis, arteritis, Alzheimer's disease, celiac disease, congestive heart failure, adult respiration distress syndrome, cerebral infarction, cerebral stroke, gout, myasthenia gravis, amyotrophic lateral sclerosis, thyroiditis, Buergur's disease, type II diabetes mellitus, obesity, Reiter's syndrome, Hodgkin's disease, liver cirrhosis, renal insufficiency, myocardial infarction, Sjoegren's syndrome, depression, eating disorders, allotriophagy, and taste disorders; and the like can be mentioned.

As other diseases and conditions that occur, exacerbate or recur with the involvement of IL-18 and cytokines released thereby, circadian rhythm abnormalities, appetite abnormalities, pituitary insufficiencies such as Addison's disease, acromegaly, and gonadal developmental disorders, which are abnormalities of the control of the nervous and endocrine systems; thyroid functional impairment, obesity, glucose control abnormalities; the secondary occurrence, recurrence or exacerbation of primary aldosteronism, Cushing's disease, and diseases pathologically characterized by joint inflammation, such as articular rheumatism, gout and arthritis, due to non-inflammatory stress such as cold or mental stress, insufficient sleep, bloodstream disorders, physical weight loading and the like; renal tubular epithelial cell loss due to infection, poisons, and anticancer agents that injure the renal tubules, such as cisplatin and carboplatin, or by an idiopathic mechanism, can be mentioned.

Although the therapeutic agent of the present invention is thought to be effective in the treatment and amelioration of all of the above-described diseases, it is more effective against autoimmune diseases, skin diseases, central nervous system diseases, various diseases caused by pituitary insufficiencies and the like, still more effective against systemic lupus erythematosus (SLE), lupus nephritis, multiple sclerosis, bronchial asthma, atopic dermatitis, psoriasis, cerebral ischemia, cerebral degeneration, encephalitis, articular rheumatism, emmeniopathy, endometriosis, sexual desire reduction and the like, and particularly effective against lupus nephritis.

Furthermore, according to the present invention, provided is a method of screening for a substance having a therapeutic effect, ameliorating effect or prophylactic effect on a disease that occurs, exacerbates or recurs due to non-inflammatory stress, or a substance having a suppressive effect or preventive effect on oxidative stress, which comprises measuring the amount or activity of the above-described indicator agent. According to the method of the present invention, screening for a substance having a therapeutic, ameliorating or prophylactic effect on a disease involved directly or indirectly by non-inflammatory stress can be performed in large amounts, quickly, and conveniently. A substance screened for by this method is effective as a therapeutic drug, ameliorating drug or prophylactic drug for a disease that occurs, exacerbates or recurs due to non-inflammatory stress. Also, according to the present screening method, a substance having a suppressive effect or preventive effect on oxidative stress can be screened for. Because superoxide is present upstream of the cytokine cascade in non-inflammatory stress, according to the present screening method, a substance having a suppressive effect or preventive effect on oxidative stress can be screened for with the aforementioned indicator agent as the index, and the present screening method is helpful in developing pharmaceuticals and foods with health claims (foods for specified health uses and foods with nutrient function claims) with such a substance as an active ingredient.

The screening method of the present invention comprises, specifically, for example, the following steps:
1) a step for culturing cultured cells under test substance presence conditions, or rearing an animal under test substance administration conditions,
2) a step for quantifying an indicator agent in a sample prepared from the aforementioned cultured cells or the aforementioned animal, and
3) a step for comparing the amount of indicator agent under test substance presence conditions or under test substance administration conditions with the amount of indicator agent under test substance absence conditions or under test substance non-administration conditions, and judging a test substance described above that has significantly reduced or increased the amount of indicator agent to be a substance having a therapeutic effect, ameliorating effect or prophylactic effect on a disease that occurs, exacerbates or recurs due to non-inflammatory stress, or to be a substance having a suppressive effect or preventive effect on oxidative stress.

As examples of animals used in the present screening method, mouse, rat, rabbit, goat, sheep, hamster, chicken and the like can be mentioned, with preference given to mouse and rat because of the ease of operation. Also, a model animal or transgenic animal for a disease that occurs, exacerbates or recurs due to non-inflammatory stress may be used. As the aforementioned cultured cells used, cells having the capability of producing a cytokine, particularly cells having the capability of producing IL-18, are preferable; for example, synovial cells, renal tubular epithelial cells, microglia, astrocytes, macrophages, keratinocytes, intestinal glandular cells and the like can be mentioned.

Also, the animal and cultured cells used may or may not be previously loaded with non-inflammatory stress. For example, when a substance having a therapeutic effect and ameliorating effect on a disease that occurs, exacerbates or recurs due to non-inflammatory stress is screened for, the cultured cells or animal may be previously loaded with non-inflammatory stress, and thereafter given a test substance, and the like. When a substance having a prophylactic effect on the aforementioned disease is screened for, a test substance may be administered and the like before the cultured cells or animal is loaded with non-inflammatory stress. Here, as the non-inflammatory stress, physical stress, chemical stress, mental stress and oxidative stress are preferable, ultraviolet irradiation stress, chemical substance exposure stress or restraint stress and oxidative stress cased thereby are more preferable, and restraint stress is particularly preferable.

As examples of the aforementioned test substance, peptides, proteins, synthetic compounds, extracts from cell or plant and the like can be mentioned, and these may be novel substances or known substances. As examples of the test substance when a substance having a suppressive effect or preventive effect on oxidative stress is screened for, various vitamins, thiol compounds, various plant-derived polyphenols, antioxidant peptides/amino acids, probucol, which is used as a therapeutic drug for hyperlipemia, captopril, which is used as a hypotensive agent, and the aforementioned superoxide suppressant can be mentioned.

As the sample prepared from cultured cells or an animal, cell culture broth, cell extracts, or blood, body fluid, tissue, cells, and excretes collected from an animal or extracts thereof, and the like can be mentioned, and blood collected from the aforementioned animal or a culture broth of the aforementioned cultured cells are preferably used.

As the aforementioned indicator agent, IL-18 and active type IL-18 are preferable, and active type IL-18 is particularly preferable. Although the indicator agent in the sample can be quantified by any conventionally known method, it is preferable to use the visualizing agent of the present invention; using these visualizing agents, this quantitation can be performed according to, for example, ELISA, Western blot analysis and the like and methods based thereon.

As the above-described disease that occurs, exacerbates or recurs due to non-inflammatory stress, the aforementioned autoimmune diseases, skin diseases, various infectious diseases, mental diseases, cardiovascular diseases, various diseases caused by pituitary insufficiencies, malignant tumors and the like can be mentioned, but the present screening method can be suitably used for screening for a substance having a therapeutic effect, ameliorating effect or prophylactic effect preferably on autoimmune diseases, skin diseases, ischemic diseases, mental diseases, cardiovascular diseases, and various diseases caused by pituitary insufficiencies, more preferably on systemic lupus erythematosus, lupus nephritis, atopic dermatitis, psoriasis, cerebral ischemia, cerebral degeneration, encephalitis, articular rheumatism, multiple sclerosis, bronchial asthma, cardiomyopathy, emmeniopathy, endometriosis, and sexual desire reduction, and particularly preferably on lupus nephritis.

When keratinocytes are used as the cultured cells, a substance having a therapeutic effect, ameliorating effect or prophylactic effect on skin disorders due to ultraviolet and the like, atopic dermatitis, psoriasis, acne vulgaris, pemphigus, ichthyosis, and photosensitivity, and on diseases involving inflammatory changes in the skin in their pathology, such as burns, radiation disorders of the skin, and wound healing, can be suitably screened for.

When microglia or astrocytes are used as the cultured cells, a substance having a therapeutic effect, ameliorating effect or prophylactic effect on brain inflammation, neuronal loss, brain insufficiencies, and diseases, for example, Alzheimer's disease, Alzheimer type senile dementia, diffuse Lewy body disease, Pick's disease, Binswanger's disease, Parkinson's disease, Parkinson syndrome, cerebral ischemia, cerebral ischemia/reperfusion disorders, carbon monoxide poisoning, thinner poisoning, neonatal hemorrhagic encephalopathy, hypoxic encephalopathy, hypertensive encephalopathy, epilepsy, multiple sclerosis, HIV encephalopathy, brain circulatory disorders, cerebrovascular disorder and the like; and a substance having a therapeutic effect, ameliorating effect or prophylactic effect on circadian rhythm abnormalities, appetite abnormalities, pituitary insufficiencies such as Addison's disease, acromegaly and gonadal developmental disorders, thyroid functional impairment, obesity, and glucose control abnormalities; and secondarily on adrenal insufficiencies such as primary aldosteronism and Cushing's disease, abnormalities in gastrointestinal tract movement regulation and bone formation, and the like, can be suitably screened for.

When macrophages are used as the cultured cells, for example, a substance having a therapeutic effect, ameliorating effect or prophylactic effect on all inflammatory diseases mediated by macrophages and IL-18 with the involvement of non-inflammatory stress, autoimmune diseases, and body defense system regulatory abnormalities, can be suitably screened for.

When synovial cells are used, a substance having a therapeutic effect, ameliorating effect or prophylactic effect on the occurrence, recurrence or exacerbation of diseases pathologically characterized by joint inflammation, such as articular rheumatism, gout and arthritis, due to non-inflammatory stress such as cold or mental stress, insufficient sleep, bloodstream disorders, and physical weight loading, can be suitably screened for.

When renal tubular epithelial cells are used, a substance having a therapeutic effect, ameliorating effect or prophylactic effect on the occurrence or exacerbation of diseases pathologically characterized by renal tubular epithelial cell loss and inflammation due to infection, poisons, and anticancer agents that injure the renal tubules, such as cisplatin and carboplatin, or by an idiopathic mechanism, can be suitably screened for.

The present screening method is, for example, performed as described below.

When cultured cells are used, the present screening method is performed by culturing cultured cells in a stressed state under test substance presence conditions, detecting and quantifying the IL-18 released from the cells into the culture broth using the visualizing agent of the present invention, comparing the amount of IL-18 released under the test substance presence conditions with the amount of IL-18 released under test substance absence conditions, and judging a test substance described above that has significantly reduced or increased the amount of IL-18 released to be a substance having a therapeutic effect, ameliorating effect or prophylactic effect on a disease that occurs, exacerbates or recurs due to non-inflammatory stress. When an animal is used, the present screening method is performed by rearing an animal in a stressed state under test substance administration conditions, measuring the concentration of IL-18 contained in the blood of the aforementioned animal, comparing this with the blood concentration of IL-18 obtained when the animal is reared under test substance non-administration conditions, and judging a test substance as described above that has significantly reduced or increased the blood concentration of IL-18 to be a substance having a therapeutic effect, ameliorating effect or prophylactic effect on a disease that occurs, exacerbates or recurs due to non-inflammatory stress.

### Examples

The present invention is hereinafter described in more detail by means of the following Examples, which, however, are not to be construed as limiting the invention.

### [Animals]

Male C57B6 mice were purchased from Seac Yoshitomi. IL-18 knockout (KO) mice (C57/B6 background, Immunity. 8, 383-390 (1998)) and caspase-1 knockout (KO) mice (Balb/C background, Science. 267, 2000-2003 (1995)) were supplied by Dr. Takeda and Dr. Kuida, respectively. MRL/MpJUmmCrj-lpr/lpr (MRL/lpr) mice were purchased from Charles River Japan Inc. The mice were housed at 20°C at 1 animal per cage. Experiments using the mice were started at 10 o'clock.

### [Reagents]

An anti-ACTH antiserum manufactured by Progen Biotechnik (Germany), an anti-mouse IL-18 monoclonal antibody manufactured by MBL (Japan), an anti-mouse caspase-1 antibody (sc-515) manufactured by Santa Cruz (USA), an anti-mouse/rat caspase-11 antibody manufactured by BIOMOL (USA), and an anti-phosphorylated (Thr180/Tyr182) P38 MAP kinase antibody manufactured by Cell Signaling Technology (USA) were used. An anti-mouse IL-6 polyclonal antibody manufactured by Chemi-Con Company was used. For an anti-mouse IL-18 receptor antibody, a partial peptide (20 amino acids) of the hydrophilic region in the immediate vicinity of the transmembrane region of mouse IL-18 receptor was synthesized according to a conventional method, and immunized to a rabbit, and the antiserum obtained was used.

Synthetic ACTH (1-24 amino acids) was purchased from Peninsula Laboratories Inc. (USA). Ac-YVAD-CHO and Ac-YVAD-MCA were purchased from Peptide Institute, Inc. Z-FA-fmk (J. Biol. Chem. 276, 21153-21157 (2000)) and SB203580 were purchased from Calbiochem-Novabiochem (USA) and BIOMOL Research Laboratories, respectively. Diphenyleneiodonium chloride (DPI) and nicotinamidoadenine dinucleotide phosphate (NADPH) oxidase inhibitors and superoxide dismutase (SOD)-1 were purchased from Sigma (USA).

### [Restraint stress]

For restraint stress, each mouse was kept in a restrainer (a round plastic tube 27 mm in diameter) for 1, 3 or 6 hours, and IL-18 levels were measured. Regarding the analysis of IL-6, each mouse was restrained as described above, and then housed in the starting cage for 6 hours, after which a measurement was performed.

### [Assay for cytokines]

After restraint, each mouse was anesthetized with diethyl ether, and blood was drawn into an EDTA-containing tube. Plasma was separated by centrifugation at 4°C and 10000 x g for 10 minutes, and stored at -80°C. IL-18 and IL-6 were analyzed by ELISA using QuantikineTM immunoassay kit (R&D Systems) and OptEIATM (BD-Pharmingen), respectively.

### [Statistical analysis]

All experimental data are shown as the mean±S.D. for three or more independent experiments. Statistical comparisons among different treatments were performed by Student's t-test using the GraphPad Prism program (GraphPad Software Inc.). A P-value less than 0.05 was considered to indicate statistical significance. The symbols shown in the figures are as follows:
*; P<0.05 versus control
**; P<0.01 versus control
***; P<0.001 versus control
#; P<0.01 (n=4-9) versus stress loading
   P<0.05 (n=4-9) versus stress loading (in case of Fig. 4)
   P<0.05 versus wild type (in case of Fig. 5) ##; P<0.001 versus stress loading (n=4-9)
   P<0.01 (n=4-9) versus stress loading (in case of Fig. 4)
   P<0.01 versus wild type (in case of Fig. 5) +; P<0.01 versus stress-loaded wild type (n=6).

### [Example 1]

### Measurement of plasma IL-18 protein levels in restraint stress mice

The anti-ACTH antiserum (50 µl, 1:1), dexamethasone (1 ng/50 µl PBS(-)) or the anti-IL-18 antibody (50 µl, 1:1) was administered intraperitoneally to each mouse; after 10 minutes elapsed, the animal was kept in a restraint tube for 6 hours. Synthetic ACTH (250 µg/50 µl PBS(-)) was injected to the left leg; after 6 hours elapsed, the mice were subjected to the experiment. The results are shown in Fig. 1.
(1) Elevation of plasma ACTH level precedes an increase in plasma IL-18 level
   From Fig. 1A, the plasma IL-18 levels in the mice exposed to restraint stress were 43±34.3 pg/ml (before stress loading), 330±64.3 pg/ml (3 hours after stress loading) and 1059±30.022 pg/ml (6 hours after stress loading). ACTH in plasma increased more rapidly than IL-18, and reached about 500 pg/ml within 1 hour after stress loading but decreased to the baseline level in 3 hours. From this fact, it is found that the elevation of plasma ACTH level precedes an increase in plasma IL-18 level. Note that an elevation of plasma ACTH level indicates the activation of the hypothalamus-pituitary-adrenal (HPA) axis.
(2) ACTH is involved in stress-induced elevation of plasma IL-18
   ACTH is known to cause the adrenal-specific expression of IL-18 mRNA. Hence, whether or not ACTH is involved in the stress-induced elevation of plasma IL-18 was examined. Mice were treated with the anti-ACTH antiserum or dexamethasone, and IL-18 levels in plasma or in the adrenal were analyzed. From Fig. 1B and Fig. 1C, it was found that the anti-ACTH antiserum and dexamethasone inhibited the stress-induced increases in IL-18 levels in plasma (Fig. 1B) and in the adrenal (Fig. 1C). In mice having one adrenal extirpated, the IL-18 levels after stress loading were lower than those in sham-operated mice. From these results, it was suggested that by stress-induced ACTH, elevations of IL-18 levels in plasma and the adrenal might be caused.
(3) ACTH first influences IL-18 protein levels in the adrenal
   When ACTH was administered, the plasma IL-18 level rose, but its degree was lower than that with stress loading (Fig. 1D). The IL-18 level in the adrenal rose to the same extent as in the case of stress loading (Fig. 1E). From these results, it is suggested that in the stress-loaded mice, ACTH mainly results in a stress-induced elevation of IL-18 in the adrenal, and that another factor might be involved in the elevation of plasma IL-18 level.
(4) Properties of IL-18 protein
   As a result of an analysis of IL-18 protein by Western blotting, it was found that IL-18 occurred as a 18-kD mature type protein in plasma (Fig. 1F, lanes 1 to 3), and on the other hand occurred mainly as a 24-kD precursor type in the adrenal (Fig. 1F, lanes 4 to 6). From these results, it is suggested that the IL-18 precursor in the adrenal may be processed and released into plasma.

### [Example 2]

### Role of caspase-1 in stress

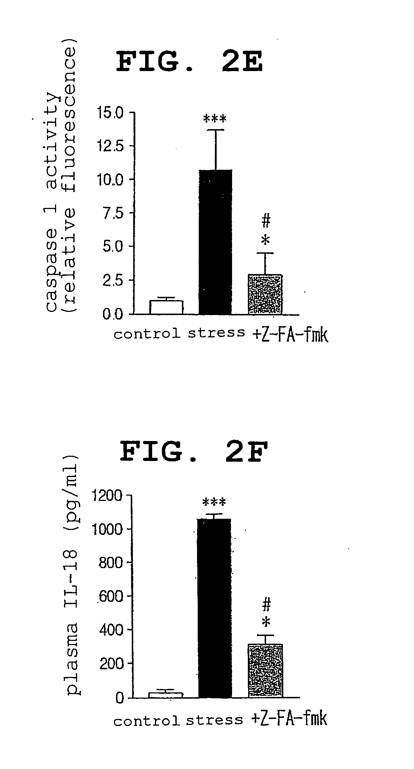

Each mouse was kept in a restrainer for 1 hour, and Ac-YVAD-CHO (200 µM/50 µl PBS(-)), Z-FA-fmk (100 µM/50 µl PBS(-)) and SB-203580 (100 µg/50 µl PBS(-) per mouse) were administered intraperitoneally. 1 and 3 hours after the start of restraint, SOD-1 (20 U) and DPI (30 µg) were administered intraperitoneally to the mouse, and samples were analyzed by Western blotting. The results are shown in Fig. 2.

Caspase-1 was induced in the adrenal by ACTH (Fig. 2A, lane 2) and stress (Fig. 2A, lane 3). Caspase-1 activity in the adrenal increased 10 fold after stress loading (Fig. 2B). The caspase-1 inhibitor YVAD-CHO effectively inhibited the stress-induced increase in plasma IL-18 level (Fig. 2C), but did not inhibit the IL-18 precursor protein level in the adrenal (Fig. 2D). In caspase-1 knockout (KO) mice, IL-18 was not detected in plasma just before and after stress loading, but after stress loading, the IL-18 precursor level increased. From these results, it is suggested that caspase-1 may be essential to the processing of the IL-18 precursor in the adrenal, and essential to the elevation of plasma IL-18 in stress-loaded mice.

### [Example 3]

### Analysis of caspase-11 and MAP kinase by Western blotting

Western blot analysis of IL-18 and phosphorylated P38MAP kinase in adrenal tissue was performed as described below. The adrenal tissue was homogenized in an immunoprecipitation (IP) buffer solution (50 mM Tris pH 7.4, 150 mM NaCl, 0.5% NP-40 and 0.5% sodium deoxycholate) containing a protease inhibitor mixture (manufactured by Roche Diagnostics) using a Polytron homogenizer. The homogenate obtained (100 µg protein) was pre-clarified using protein A/G agarose (Exalpha Biologicals, Inc.), incubated with the anti-mouse IL-18 monoclonal antibody or the anti-phosphorylated (Thr180/Tyr182) P38 MAP kinase antibody and protein A/G agarose (50 µl), and centrifuged (at 13000 x g for 25 minutes, 4°C). The precipitate was boiled, and the adrenal protein was recovered, electrophoresed in 10-20% SDS-polyacrylamide gradient gel, and electrically transferred to a Hybond ECL (manufactured by Amersham) nitrocellulose membrane. The aforementioned membrane was incubated with an antibody (IL-18, phosphorylated (Thr180/Tyr182) P38 MAP kinase, caspase-1 or caspase-11), and the reacted protein was detected using an ECL plus system (manufactured by Amersham biosciences). Procaspase-11 and activated P38 MAP kinase were quantified using a Science Lab 99 image analysis system (manufactured by Fujifilm).
(1) Involvement of caspase-11 in the upregulation of caspase-1 activity
   Since caspase-1 is reported to be activated by caspase-11, the effect of the caspase-11 inhibitor Z-FA-fmk on plasma IL-18 level was examined. The results are shown in Figs. 2E and 2F.
   It was found that Z-FA-fmk suppressed the stress-induced increase in caspase-1 activity in the adrenal (Fig. 2E), and suppressed plasma IL-18 level (Fig. 2F). From this fact, it is suggested that caspase-11 may be involved in the stress-induced activation of caspase-1 in the adrenal.
(2) Involvement of P38MAP kinase in the upregulation of caspase-1 activity
   It has been reported that the induction of caspase-11 by LPS requires the activation of NF-κB, and that the activation of NF-κB is mediated by P38MAP kinase. In vitro, it has been reported that the induction of caspase-11 is suppressed by the P38MAP kinase inhibitor SB203580. In the present invention, in vivo, that is, in stress-loaded mice, the effect of SB203580 on the induction of caspase-11 was examined. The results are shown in Fig. 3.
   SB203580 inhibited the stress-induced increase in procaspase-11 protein (Fig. 3A). In the SB203580-treated mice, caspase-1 activity in the adrenal (Fig. 3B) and plasma IL-18 level were also lower than those in control mice (Fig. 3C). With the treatment using SB203580 and Z-FA-fmk in combination, no further reduction in the activity of caspase-1 (Fig. 3B), plasma IL-18 level (Fig. 3C) and 24-kD IL-18 precursor level in the adrenal (not illustrated) did not occur. Neither YVAD-CHO nor Z-FA-fmk influenced the phosphorylation of P38MAP kinase in the adrenal (Fig. 3D). The phosphorylation of P38MAP kinase, like in wild type mice (Fig. 3D, lane 2), was induced by stress in caspase-1 KO mice (Fig. 3D, lane 5). Therefore, this phosphorylation did not depend on caspase-1.

### [Example 4]

### Localization of IL-18, caspase-1, caspase-11 and P38MAP kinase by immunohistochemistry

The adrenal was fixed in formalin, embedded in paraffin, and cut into 4-µm sections. The aforementioned tissue sections were incubated with an antibody (P38 MAP kinase, caspase-11, caspase-1 (these three were diluted 200 fold) or IL-18 (diluted 400 fold)). Using a horseradish peroxidase-conjugated secondary antibody (manufactured by Santa Cruz) and diaminobenzidin (DBA), color development was performed. For control, sections without the primary antibody and sections derived from a mouse without stress were used. The results are shown in Figs. 3E and 3F.

In the stress-loaded mice, IL-18, caspase-1, caspase-11 and activated P38MAP kinase were co-localized in the zona reticularis of adrenal cortex (Fig. 3E), whereas in the control mice, none of them was detected. An inhibitor of caspase-1 (Fig. 3F, panel 1) and an inhibitor of caspase-11 (Fig. 3F, panel 2) did not influence the accumulation of phosphorylated P38MAP kinase in adrenal cortex.

From the results of Example 3(2) and Example 4 above, it is shown that P38MAP kinase is involved in the stress-induced elevation of plasma IL-18 level via the induction of caspase-11, which in turn activates caspase-1.

### [Example 5]

### Analysis of caspase-1 activity

Caspase-1 activity in the adrenal was measured using a caspase assay kit (manufactured by BD-Pharmingen). Describing briefly, a homogenate of adrenal tissue was incubated with Ac-YVAD-MCA (acetyl-L-tyrosyl- L-valyl- L-alanyl- L-aspartic acid 4-methyl-coumaryl-7-amide), which is a fluorescent substrate for caspase-1, and the MCA leaving from the aforementioned substrate was measured using a fluorescence plate reader (excitation wavelength 380 nm, irradiation wavelength 420-460 nm).

### [Example 6]

### Involvement of superoxide in the upregulation of P38MAP kinase

Active oxygen species are reported to mediate the phosphorylation of P38MAP kinase in pulmonary fibroblasts, neutrophils and monocytes. In stress-loaded mice, the effect of SOD on the phosphorylation of P38MAP kinase was examined. The results are shown in Fig. 4.

It is found that SOD inhibits the phosphorylation of P38MAP kinase by about 70% in the adrenal (Fig. 4A). Also, SOD, in the stress-loaded mice, also reduced the levels of procaspase-11 (Fig. 4B), caspase-1 activity (Fig. 4C) and plasma IL-18 (Fig. 4D), but did not reduce the adrenal level of IL-18 precursor protein (Fig. 4E). Treatment with SOD in combination with SB203580, Z-FA-fmk or YVAD-CHO did not result in a further reduction in the level of procaspase-11, caspase-1 or plasma IL-18, respectively. The induction of plasma IL-18 by stress was suppressed by DPI, that is, an NADPH oxidase inhibitor (Fig. 4F). This supports the notation that a superoxide anion is involved in the induction of plasma IL-18 by stress via the phosphorylation of P38MAP kinase.

### [Example 7]

### Involvement of IL-18 in the control of plasma IL-6 level in stress-loaded mice

Using IL-18-deficient mice, the relationship between IL-6, which is another stress-related cytokine, and IL-18, was examined. Without stress, IL-6 was undetectable in WT and IL-18 KO, but after 3 hours of stress loading, the IL-6 level was 230 pg/ml in WT and 25 pg/ml in IL-18 KO (Fig. 5A). After liberation from the stress, plasma IL-6 was kept at about 80 pg/ml in WT, but decreased to 5 pg/ml in IL-18 KO (Fig. 5A). Treatment with YVAD-CHO, SOD and anti-IL-18 antibody 70% inhibited the stress-induced increase in plasma IL-6 level in WT (Fig. 5B). From these results, it is suggested that stress may induce IL-6 in plasma IL-18-dependently.

### [Example 8]

### Treatment of lupus nephritis by administration of IL-18 receptor antibody

MRL/lpr mice at 10 weeks of age were loaded with 3 hours of restraint stress, and concentrations of IL-6 in plasma were examined. Also, 10 minutes before stress loading, the anti-IL-18 receptor antibody was administered to the aforementioned mice, and changes in the concentration of IL-6 were examined. As a result, at 6 hours after stress loading, the concentration of IL-18 in plasma rose to 1132, 882 and 793 pg/ml, and the elevation of the concentration of IL-6 in plasma reached a peak 3 hours after stress loading. In the MRL/lpr mice after stress loading, an elevation of IL-6 in plasma was observed, but in the mice not loaded with stress, IL-6 was not detected. When mice receiving the anti-IL-18 receptor antibody were loaded with stress, the elevation of IL-6 was suppressed.

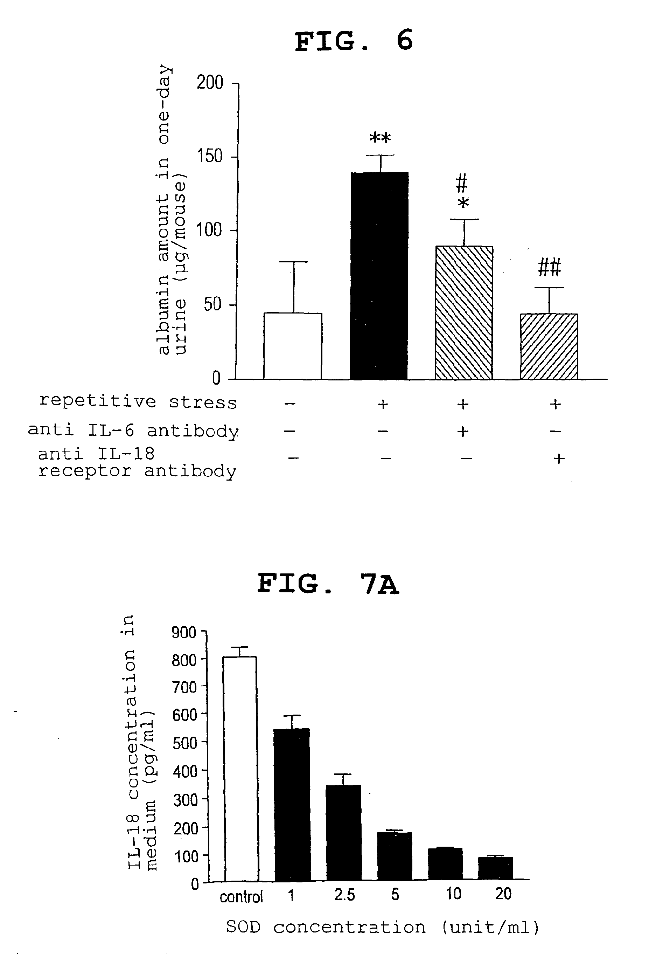

MRL/lpr mice have spontaneously developing lupus nephritis. MRL/lpr mice at 12 weeks of age were loaded with 6 hours of restraint stress every 2 days (3 times/week) for 3 weeks. The aforementioned mice were divided into a group with stress only, a group receiving the anti-IL-6 antibody, and a group receiving the anti-IL-18 receptor antibody, and albumin contents in one-day pools of urine (proteinuria) were examined. For control, MRL/lpr mice not loaded with stress were also examined. The results are shown in Fig. 6.

From Fig. 6, the MRL/lpr mice experienced increased proteinuria and exacerbated lupus nephritis when loaded with stress. However, it is found that by administering the anti-IL-6 antibody or the anti-IL-18 receptor antibody, the MRL/lpr mice have the stress-induced exacerbation of lupus nephritis suppressed. Particularly, administration of the anti-IL-18 receptor antibody was effective.

### [Example 9]

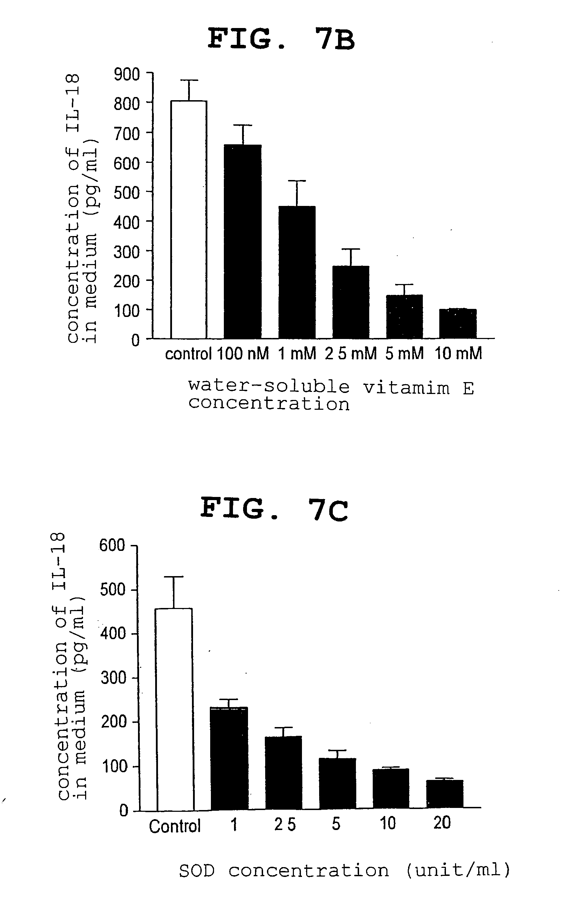

Primary culture human skin keratinocytes (keratinocytes) were cultured in a basal medium for keratinocytes contained in petri dishes for cell culture 6 cm in diameter at 1.0-2.0 x 10⁶/dish. This was cultured in the aforementioned medium containing 1 µM of paraquat, which is an active oxygen generator, for 15 minutes, after which the medium was exchanged with a paraquat-free medium, and the cells were further cultured for 1 hour, after which the medium was collected, and the concentration of IL-18 protein in the medium was measured. Also, during paraquat treatment, the cells were cultured in a medium containing SOD (1, 2.5, 5, 10, 20 U/ml), which is an active oxygen eliminator, or water-soluble vitamin E (100 nM, 1, 2.5, 5,10 mM/ml), and the concentration of IL-18 protein in the medium was measured. In the keratinocytes, the activation and release of IL-18 by the active oxygen molecular species were observed. Furthermore, these were suppressed by SOD, which is an active oxygen eliminator, or by vitamin E, which neutralizes active oxygen, dose-dependently (Figs. 7A and 7B).

Primary culture human skin keratinocytes (keratinocytes: purchased from CAMBREX Company) were cultured in a basal medium for keratinocytes contained in petri dishes for cell culture 6 cm in diameter at 1.0-2.0 x 10⁶/dish. This was irradiated with 100 mJ of ultraviolet B rays (UV-B rays), after which the cells were cultured for 1 hour, after which the medium was collected, and the concentration of IL-18 protein in the medium was measured. Also, after the UV-B ray treatment, the cells were cultured in a medium containing SOD (1, 2.5, 5, 10, 20 U/ml), which is an active oxygen eliminator, or water-soluble vitamin E (100 nM, 1, 2.5, 5, 10 mM/ml), and the concentration of IL-18 protein in the medium was measured. In the keratinocytes, the activation and release of IL-18 by the active oxygen molecular species were observed. Furthermore, these were suppressed by SOD, which is an active oxygen eliminator, or by vitamin E, which neutralizes active oxygen, dose-dependently (Figs. 7C and 7D).

### [Example 11]

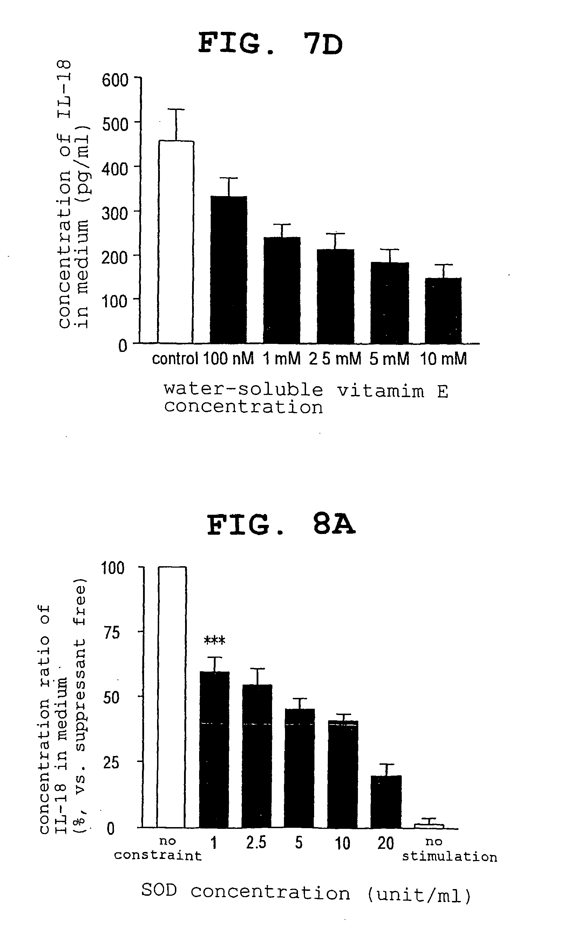

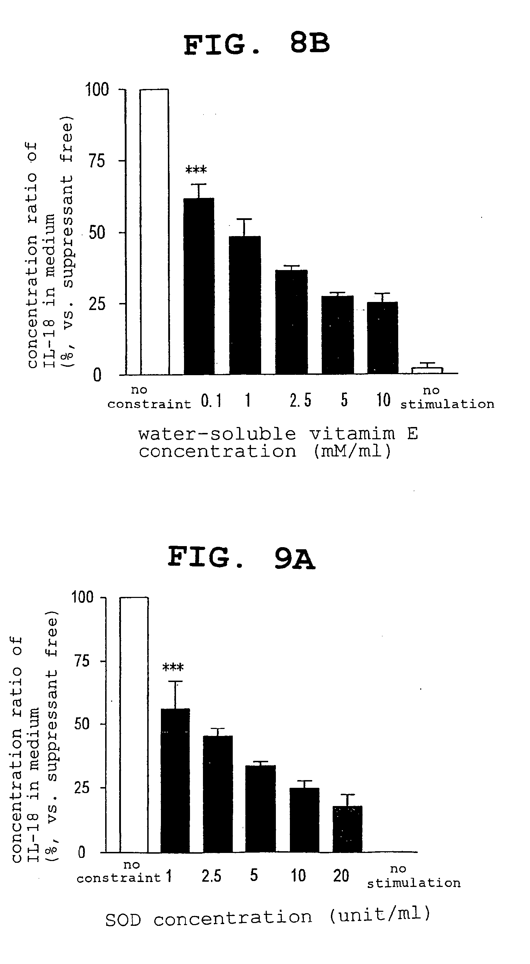

Brain microglia isolated from the mouse neonatal brain by the method of Hassan et al. (Hassan NF, Rifat S, Campbell DE, McCawley LJ, Douglas SD. J. Leukoc Biol. 1991 Jul; 50(1):86-92) were cultured at 1.5-2.0 x 10⁶/ml. This was cultured in Dulbecco's minimum essential medium containing 100 nM of paraquat, which is an active oxygen generator, for 1 hour, after which the medium was exchanged with a paraquat-free medium. After further cultivation for 3 hours, the medium was collected, and the concentration of IL-18 protein in the medium was measured. Also, after the paraquat treatment, the cells were cultured in a medium containing SOD (1, 2.5, 5, 10, 20 U/ml), which is an active oxygen eliminator, or water-soluble vitamin E (100 nM, 1, 2.5, 5, 10 mM/ml), and the concentration of IL-18 protein in the medium was measured. In the microglia, the activation and release of IL-18 by the active oxygen molecular species were observed. Furthermore, these were suppressed by SOD, which is an active oxygen eliminator, or by vitamin E, which neutralizes active oxygen, dose-dependently (Figs. 8A and 8B).

### [Example 12]

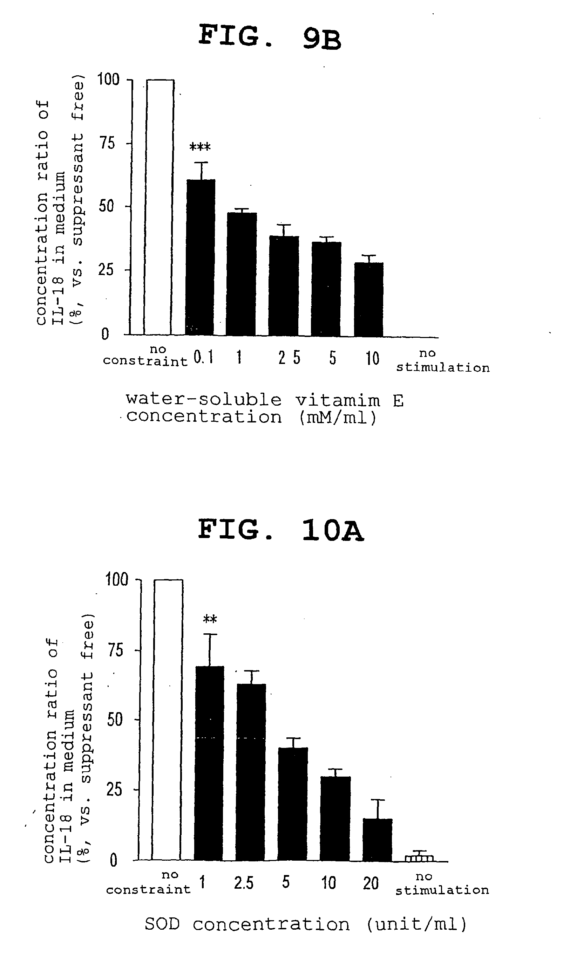

Brain astrocytes isolated from the mouse neonatal brain by the method of Yang et al. (Yang P, Hernandez MR. Brain Res Brain Res Protoc. 2003 Oct; 12(2):67-76) were prepared at 1.5-2.0 x 10⁶/ml. This was cultured in Dulbecco's minimum essential medium containing 100 nM of paraquat, which is an active oxygen generator, for 1 hour, after which the medium was exchanged with a paraquat-free medium. The cells were further cultured for 3 hours, after which the medium was collected, and the concentration of IL-18 protein in the medium was measured. Also, after the paraquat treatment, the cells were cultured in a medium containing SOD (1, 2.5, 5, 10, 20 U/ml), which is an active oxygen eliminator, or water-soluble vitamin E (100 nM, 1, 2.5, 5, 10 mM/ml), and the concentration of IL-18 protein in the medium was measured. In the astrocytes, the activation and release of IL-18 by the active oxygen molecular species were observed. Furthermore, these were suppressed by SOD, which is an active oxygen eliminator, or by vitamin E, which neutralizes active oxygen, dose-dependently (Figs. 9A and 9B).

### [Example 13]

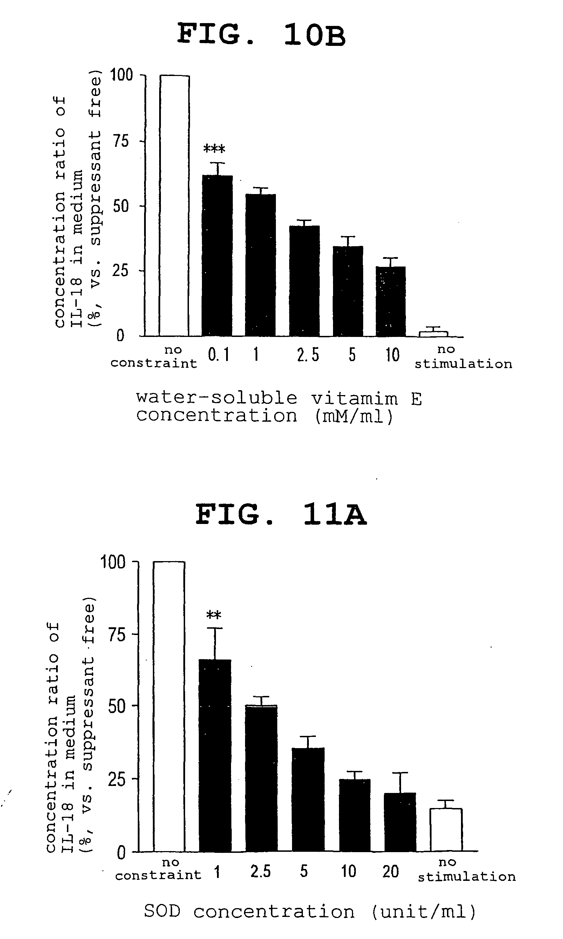

Abdominal macrophages isolated from mice by the method of Hamilton et al. (Hamilton TA, Weiel JE, Adams DO., J Immunol. 1984 May; 132(5):2285-90) were cultured in Dulbecco's minimum essential medium at 1.0-2.0 x 10⁵/dish. The medium was exchanged with the aforementioned medium containing 100 nM of paraquat, which is an active oxygen generator, and the cells were cultured for 15 minutes, after which the medium was exchanged with a paraquat-free medium, and the cells were further cultured for 3 hours, after which the medium was collected, and the concentration of IL-18 protein in the medium was measured. Also, during paraquat treatment, the cells were cultured in a medium containing SOD (1, 2.5, 5, 10, 20 U/ml), which is an active oxygen eliminator, and the concentration of IL-18 protein in the medium was measured. In the macrophages, the activation and release of IL-18 by the active oxygen molecular species were observed. Furthermore, these were suppressed by SOD, which is an active oxygen eliminator, or by vitamin E, which neutralizes active oxygen, dose-dependently (Figs. 10A and 10B).

### [Example 14]

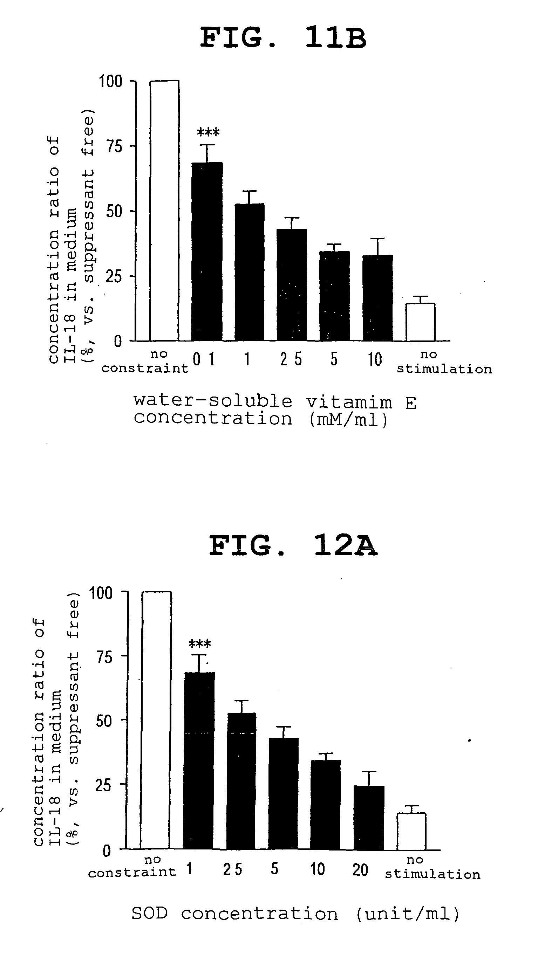

Synovial membrane tissue collected from the articular synovial membrane at a lesion site of an articular rheumatism patient was subjected to tissue culture in an RPMI-1640 medium containing 10% fetal bovine serum by the method of Tanaka et al. (Tanaka M, Harigai M, Kawaguchi Y, Ohta S, Sugiura T, Takagi K, Ohsako-Higami S, Fukasawa C, Hara M, Kamatani N., J Rheumatol. 2001 Aug; 28(8):1779-87). The medium was exchanged with the aforementioned medium containing 100 nM of paraquat, which is an active oxygen generator, and the cells were cultured for 15 minutes, after which the medium was exchanged with a paraquat-free medium, and the cells were further cultured for 1 hour, after which the medium was collected, and the concentration of IL-18 protein in the medium was measured. Also, during paraquat treatment, the cells were cultured in a medium containing SOD (1, 2.5, 5, 10, 20 U/ml), which is an active oxygen eliminator, and the concentration of IL-18 protein in the medium was measured. In the synovial membrane tissue, the activation and release of IL-18 by the active oxygen molecular species were observed. Furthermore, these were suppressed by SOD, which is an active oxygen eliminator, or by vitamin E, which neutralizes active oxygen, dose-dependently (Figs. 11A and 11B).

### [Example 15]

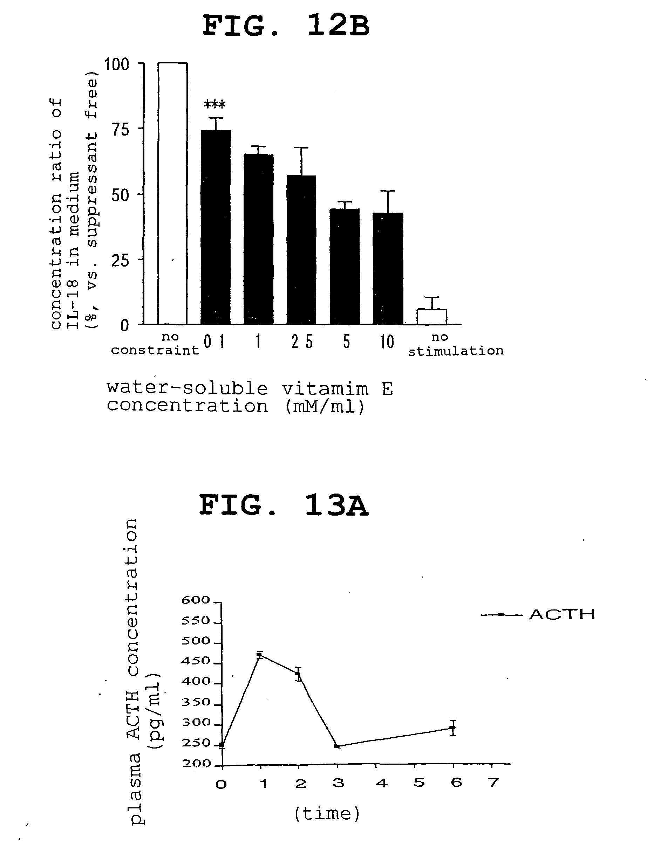

Primary culture human renal proximal tubular epithelial cells (purchased from CAMBREX Company) were cultured in a basal medium for renal tubular cells: contents 10 µg/ml hEGF (recombinant human epithelial cell growth factor) 0.5 ml, 5 mg/ml insulin 0.5 ml, 0.5 mg/ml hydrocortisone 0.5 ml, FBS (fetal bovine serum) 2.5 ml, 0.5 mg/ml epinephrine 0.5 ml, 6.5 µg/ml triiodothyronine 0.5 ml, 10 mg/ml transferrin 0.5 ml, 50 mg/ml gentamycin, 50 µg/ml amphotericin-B 0.5 ml), in petri dishes for cell culture 6 cm in diameter at 1.0-2.0 x 10⁶/dish. This was cultured in the aforementioned medium containing 100 nM of paraquat, which is an active oxygen generator, for 15 minutes, after which the medium was exchanged with the same but paraquat-free medium. After the cells were further cultured for 1 hour, the medium was collected, and the concentration of IL-18 protein in the medium was measured. Also, during paraquat treatment, the cells were cultured in a medium containing SOD (1, 2.5, 5, 10, 20 U/ml), which is an active oxygen eliminator, or water-soluble vitamin E (100 nM, 1, 2.5, 5, 10 mM/ml), and the concentration of IL-18 protein in the medium was measured. In the renal tubular cells, the activation and release of IL-18 by the active oxygen molecular species were observed. Furthermore, these were suppressed by SOD, which is an active oxygen eliminator, or by vitamin E, which neutralizes active oxygen, dose-dependently (Figs. 12A and 12B).

### [Example 16]

### IL-18 elicits the release of ACTH, FSH, and LH

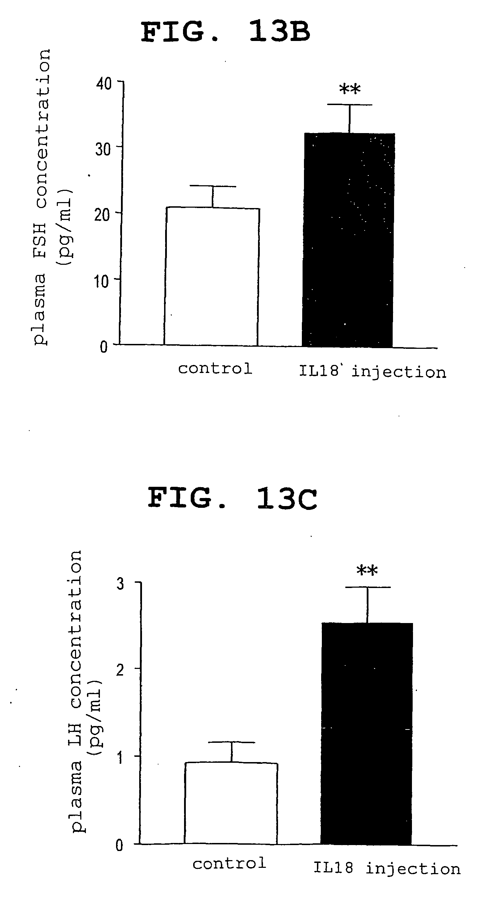

1.5 µg of IL-18 prepared by genetic recombination technology was intravenously injected to C57B/6 mice, and thereafter ACTH, FSH and LH levels in blood were examined. As shown in Fig. 13A, the blood ACTH concentration rose with the administration of IL-18. Figs. 13B and 13C show FSH and LH levels in plasma collected from mice 1 hour after IL-18 administration. It was shown that the concentrations of FSH and LH in plasma rose with the administration of IL-18. From this fact, it is understood that due to an elevation of IL-18 in blood, changes occur in the blood levels of ACTH, FSH, and LH, and it is found that an elevation of blood IL-18 due to stress and active oxygen significantly affects the systemic condition.

### Industrial Applicability

The present invention relates to an indicator agent, visualizing agent, and therapeutic agent for non-inflammatory stress responses, and use thereof, and is applicable to a measurement of the degree of non-inflammatory stress which a living organism undergoes, prophylaxis, amelioration or prediction of a change in immune status based on a non-inflammatory stress response, prophylaxis or treatment of a disease that occurs, exacerbates or recurs due to non-inflammatory stress, screening for a substance having a therapeutic effect, ameliorating effect or prophylactic effect on a disease that occurs, exacerbates or recurs due to non-inflammatory stress, or screening for a substance having a suppressive effect or preventive effect on oxidative stress, and the like.

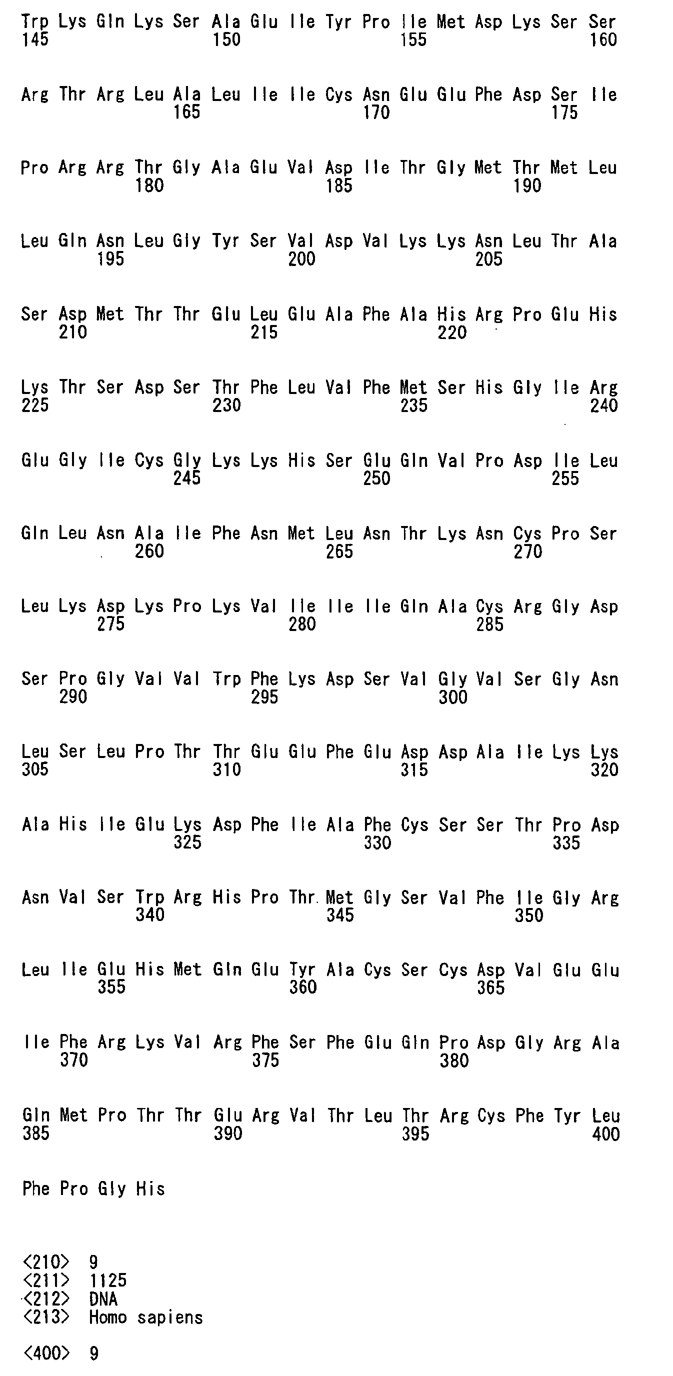

## Claims

1. An indicator agent for superoxide-mediated non-inflammatory stress responses, which comprises an ingredient involved in the non-inflammatory stress cascade.

2. The indicator agent of claim 1, wherein the ingredient involved in the non-inflammatory stress cascade contains 1 or 2 or more proteins selected from the group consisting of IL-18, active type IL-18, P38MAP kinase, phosphorylated P38MAP kinase, caspase 11, caspase 1, and activated caspase 1.

3. The indicator agent of claim 1, wherein the ingredient involved in the non-inflammatory stress cascade is IL-18.

4. The indicator agent of claim 3, wherein the IL-18 is active type IL-18 present in blood.

5. The indicator agent of claim 3 or 4, which further comprises P38MAP kinase.

6. The indicator agent of claim 5, wherein the P38MAP kinase is phosphorylated kinase.

7. The indicator agent of claim 6, wherein the P38MAP kinase is derived from a mouse, and the phosphorylation site of the kinase is Thr180 or Tyr182.

8. The indicator agent of claim 6, wherein the P38MAP kinase is derived from a human, and the phosphorylation site of the kinase is Thr185 or Tyr187.

9. The indicator agent of any one of claims 3 to 8, which further comprises caspase-11.

10. The indicator agent of any one of claims 3 to 9, which further comprises caspase-1.

11. The indicator agent of claim 10, wherein the caspase-1 is activated caspase-1.

12. The indicator agent of any one of claims 3 to 11, wherein the non-inflammatory stress is mental stress.

13. The indicator agent of claim 12, wherein the mental stress is restraint stress.

14. A visualizing agent for non-inflammatory stress responses for detecting the indicator agent of any one of claims 1 to 13.

15. The visualizing agent of claim 14, which comprises an IL-18 antibody.

16. The visualizing agent of claim 15, wherein the IL-18 antibody specifically recognizes active type IL-18.

17. The visualizing agent of claim 15 or 16, which further comprises a P38MAP kinase antibody.

18. The visualizing agent of claim 17, wherein the P38MAP kinase antibody is an anti-phosphorylated P38MAP kinase antibody.

19. The visualizing agent of claim 18, wherein the anti-phosphorylated P38MAP kinase antibody recognizes a P38MAP kinase having phosphorylated Thr180 or Tyr182, or a P38MAP kinase having phosphorylated Thr185 or Tyr187.

20. The visualizing agent of any one of claims 15 to 19, which further comprises a caspase-11 antibody.

21. The visualizing agent of any one of claims 15 to 20, which further comprises a caspase-1 antibody or a caspase 1 substrate.

22. The visualizing agent of claim 21, wherein the caspase-1 antibody specifically recognizes activated caspase-1.

23. A method of measuring the degree of non-inflammatory stress, which comprises measuring the amount or activity of the indicator agent of any one of claims 1 to 13.

24. The method of claim 23, wherein the indicator agent is IL-18 or active type IL-18.

25. The method of claim 23 or 24, which comprises using the visualizing agent of any one of claims 14 to 22.

26. The method of any one of claims 23 to 25, wherein the non-inflammatory stress is mental stress.

27. The method of claim 26, wherein the mental stress is restraint stress.

28. An assay kit for non-inflammatory stress, which comprises the visualizing agent of any one of claims 14 to 22.

29. The kit of claim 28, wherein the visualizing agent comprises an IL-18 antibody.

30. The kit of claim 29, wherein the IL-18 antibody recognizes active type IL-18.

31. A method of preventing, ameliorating or predicting a change in immune status based on a non-inflammatory stress response, which comprises applying the visualizing agent of any one of claims 14 to 22 to an animal.

32. The preventing, ameliorating or predicting method of claim 31, wherein the animal is a vertebral animal other than a human.

33. A therapeutic agent for a change in immune status based on a non-inflammatory stress response for reducing the amount or activity of the indicator agent of any one of claims 1 to 13.

34. The therapeutic agent of claim 33, wherein the active ingredient of the therapeutic agent is an IL-18 antibody or an anti-IL-18 receptor antibody.

35. The therapeutic agent of claim 34, wherein the active ingredient is an IL-18 antibody, and the IL-18 antibody binds specifically to active type IL-18.

36. The therapeutic agent of claim 33, wherein the active ingredient of the therapeutic agent is superoxide dismutase or vitamin E.

37. A method of screening for a substance having a therapeutic effect, ameliorating effect or prophylactic effect on a disease that occurs, exacerbates or recurs due to non-inflammatory stress, which comprises measuring the amount or activity of the indicator agent of any one of claims 1 to 13.

38. The method of claim 37, wherein the indicator agent is IL-18 or active type IL-18.

39. The method of claim 37 or 38, wherein the disease is systemic lupus erythematosus, lupus nephritis, atopic dermatitis, multiple sclerosis, bronchial asthma, psoriasis, cerebral ischemia, cerebral degeneration, encephalitis, articular rheumatism, emmeniopathy, endometriosis or sexual desire reduction.

40. A method of screening for a substance having a suppressive effect or preventive effect on oxidative stress, which comprises measuring the amount or activity of the indicator agent of any one of claims 1 to 13.


SEQUENCE LISTING

<110>  Sekiyama, Atsuo

<120>  An Indicator of Non-inflammatory Stress Response and Use Thereof

<130>  PCT0523ZZ

<150> JP 2004-193712
<151> 2004-6-30

<150> JP 2005-023978
<151> 2005-1-31

<160>  10

<170>  PatentIn version 3.2

<210>  1
<211>  1145
<212>  DNA
<213>  Homo sapiens

<400>  1
attctctccc cagcttgctg agccctttgc tcccctggcg actgcctgga cagtcagcaa      60

ggaattgtct cccagtgcat tttgccctcc tggctgccaa ctctggctgc taaagcggct     120

gccacctgct gcagtctaca cagcttcggg aagaggaaag gaacctcaga ccttccagat     180

cgcttcctct cgcaacaaac tatttgtcgc aggaataaag atggctgctg aaccagtaga     240

agacaattgc atcaactttg tggcaatgaa atttattgac aatacgcttt actttatagc     300

tgaagatgat gaaaacctgg aatcagatta ctttggcaag cttgaatcta aattatcagt     360

cataagaaat ttgaatgacc aagttctctt cattgaccaa ggaaatcggc ctctatttga     420

agatatgact gattctgact gtagagataa tgcaccccgg accatattta ttataagtat     480

gtataaagat agccagccta gaggtatggc tgtaactatc tctgtgaagt gtgagaaaat     540

ttcaactctc tcctgtgaga acaaaattat ttcctttaag gaaatgaatc ctcctgataa     600

catcaaggat acaaaaagtg acatcatatt ctttcagaga agtgtcccag gacatgataa     660

taagatgcaa tttgaatctt catcatacga aggatacttt ctagcttgtg aaaaagagag     720

agaccttttt aaactcattt tgaaaaaaga ggatgaattg ggggatagat ctataatgtt     780

cactgttcaa aacgaagact agctattaaa atttcatgcc gggcgcagtg gctcacgcct     840

gtaatcccag ccctttggga ggctgaggcg ggcagatcac cagaggtcag gtgttcaaga     900

ccagcctgac caacatggtg aaacctcatc tctactaaaa atacaaaaaa ttagctgagt     960

gtagtgacgc atgccctcaa tcccagctac tcaagaggct gaggcaggag aatcacttgc    1020

actccggagg tagaggttgt ggtgagccga gattgcacca ttgcgctcta gcctgggcaa    1080

caacagcaaa actccatctc aaaaaataaa ataaataaat aaacaaataa aaaattcata    1140

atgtg                                                                1145

<210>  2
<211>  193
<212>  PRT
<213>  Homo sapiens

<400>  2

Met Ala Ala Glu Pro Val Glu Asp Asn Cys Ile Asn Phe Val Ala Met
1               5                   10                  15

Lys Phe Ile Asp Asn Thr Leu Tyr Phe Ile Ala Glu Asp Asp Glu Asn
            20                  25                  30

Leu Glu Ser Asp Tyr Phe Gly Lys Leu Glu Ser Lys Leu Ser Val Ile
        35                  40                  45

Arg Asn Leu Asn Asp Gln Val Leu Phe Ile Asp Gln Gly Asn Arg Pro
    50                  55                  60

Leu Phe Glu Asp Met Thr Asp Ser Asp Cys Arg Asp Asn Ala Pro Arg
65                  70                  75                  80

Thr Ile Phe Ile Ile Ser Met Tyr Lys Asp Ser Gln Pro Arg Gly Met
                85                  90                  95

Ala Val Thr Ile Ser Val Lys Cys Glu Lys Ile Ser Thr Leu Ser Cys
            100                 105                 110

Glu Asn Lys Ile Ile Ser Phe Lys Glu Met Asn Pro Pro Asp Asn Ile
        115                 120                 125

Lys Asp Thr Lys Ser Asp Ile Ile Phe Phe Gln Arg Ser Val Pro Gly
    130                 135                 140

His Asp Asn Lys Met Gln Phe Glu Ser Ser Ser Tyr Glu Gly Tyr Phe
145                 150                 155                 160

Leu Ala Cys Glu Lys Glu Arg Asp Leu Phe Lys Leu Ile Leu Lys Lys
                165                 170                 175

Glu Asp Glu Leu Gly Asp Arg Ser Ile Met Phe Thr Val Gln Asn Glu
            180                 185                 190

Asp

<210>  3
<211>  1514
<212>  DNA
<213>  Homo sapiens

<400>  3
gcccctccct ccgcccgccc gccggcccgc ccgtcagtct ggcaggcagg caggcaatcg      60

gtccgagtgg ctgtcggctc ttcagctctc ccgctcggcg tcttccttcc tcctcccggt     120

cagcgtcggc ggctgcaccg gcggcggcgc agtccctgcg ggaggggcga caagagctga     180

gcggcggccg ccgagcgtcg agctcagcgc ggcggaggcg gcggcggccc ggcagccaac     240

atggcggcgg cggcggcggc gggcgcgggc ccggagatgg tccgcgggca ggtgttcgac     300

gtggggccgc gctacaccaa cctctcgtac atcggcgagg gcgcctacgg catggtgtgc     360

tctgcttatg ataatgtcaa caaagttcga gtagctatca agaaaatcag cccctttgag     420

caccagacct actgccagag aaccctgagg gagataaaaa tcttactgcg cttcagacat     480

gagaacatca ttggaatcaa tgacattatt cgagcaccaa ccatcgagca aatgaaagat     540

gtatatatag tacaggacct catggaaaca gatctttaca agctcttgaa gacacaacac     600

ctcagcaatg accatatctg ctattttctc taccagatcc tcagagggtt aaaatatatc     660

cattcagcta acgttctgca ccgtgacctc aagccttcca acctgctgct caacaccacc 720
tgtgatctca agatctgtga ctttggcctg gcccgtgttg cagatccaga ccatgatcac 780
acagggttcc tgacagaata tgtggccaca cgttggtaca gggctccaga aattatgttg 840
aattccaagg gctacaccaa gtccattgat atttggtctg taggctgcat tctggcagaa 900
atgctttcta acaggcccat ctttccaggg aagcattatc ttgaccagct gaaccacatt 960
ttgggtattc ttggatcccc atcacaagaa gacctgaatt gtataataaa tttaaaagct 1020
aggaactatt tgctttctct tccacacaaa aataaggtgc catggaacag gctgttccca 1080
aatgctgact ccaaagctct ggacttattg gacaaaatgt tgacattcaa cccacacaag 1140
aggattgaag tagaacaggc tctggcccac ccatatctgg agcagtatta cgacccgagt 1200
gacgagccca tcgccgaagc accattcaag ttcgacatgg aattggatga cttgcctaag 1260
gaaaagctca aagaactaat ttttgaagag actgctagat tccagccagg atacagatct 1320
taaatttgtc aggtacctgg agtttaatac agtgagctct agcaagggag gcgctgcctt 1380
ttgtttctag aatattatgt tcctcaaggt ccattatttt gtattctttt ccaagctcct 1440
tattggaagg tattttttta aatttagaat taaaaattat ttagaaaaaa aaaaaaaaaa 1500
aaaaaaaaaa aaaa 1514

<210> 4
<211> 360
<212> PRT
<213> Homo sapiens

<400> 4

<210>  5
<211>  1340
<212>  DNA
<213>  Mus musculus

<400>  5
gtgcgaaaga actgaggctt tttctcatgg ctgaaaacaa acaccctgac aaaccactta      60

aggtgttgga acagctgggc aaagaagtcc ttacggagta cctagaaaaa ttagtacaaa     120

gcaatgtact gaaattaaag gaggaagata aacaaaaatt taacaatgct gaacgcagtg     180

acaagcgttg ggtttttgta gatgccatga aaaagaaaca cagcaaagta ggtgaaatgc     240

ttctccagac attcttcagt gtggacccag gcagccacca tggtgaagct aatctggaaa     300

tggaggaacc agaagaatca ttgaacactc tcaagctttg ttcccctgaa gagttcacaa     360

ggctttgcag agaaaagaca caagaaattt acccaataaa ggaggccaat ggccgtacac     420

gaaaggctct tatcatatgc aatacagagt tcaaacatct ctcactgagg tatggggcta     480

actttgacat cattggtatg aaaggccttc ttgaagactt aggctacgat gtggtggtga 540
aagaggagct tacagcagag ggcatggagt cagagatgaa agactttgct gcactctcag 600
aacaccagac atcagacagc acattcctgg tgctaatgtc tcatggcaca ctgcatggca 660
tttgtggaac aatgcacagt gaaaaaactc cagatgtgct acagtatgat accatctatc 720
agatattcaa caattgccac tgtccaggtc tacgagacaa acccaaagtc atcattgtgc 780
aggcctgcag aggtgggaac tctggagaaa tgtggatcag agagtcttca aaaccccagt 840
tgtgcagagg tgtagatcta cctaggaata tggaagctga tgctgtcaag ctgagccacg 900
tggagaagga cttcattgcc ttctactcta caaccccaca tcacttgtcc taccgagaca 960
aaacaggagg ctcttacttc atcactagac tcatttcctg cttccggaaa catgcttgct 1020
cttgtcatct ctttgatata ttcctgaagg tgcaacaatc atttgaaaag gcaagtattc 1080
attcccagat gcccaccatt gatcgggcaa ccttgacgag atatttctac ctctttcctg 1140
gcaactgaga acaaagcaac aagcaactga atctcatttc ttcagcttga agaagtgatc 1200
ttggccaagg atcacattct attcctgaaa ttccagaact agtgaaatta aggaaagaat 1260
acttatgaat tcaagaccag cctaagcaac acagtgggat tctgttccat agacaagcaa 1320
acaagcaaaa ataaaaaaaa 1340

<210> 6
<211> 373
<212> PRT
<213> Mus musculus

<400> 6

Met Ala Glu Asn Lys His Pro Asp Lys Pro Leu Lys Val Leu Glu Gln
1               5                   10                  15

Leu Gly Lys Glu Val Leu Thr Glu Tyr Leu Glu Lys Leu Val Gln Ser
            20                  25                  30

Asn Val Leu Lys Leu Lys Glu Glu Asp Lys Gln Lys Phe Asn Asn Ala
        35                  40                  45

Glu Arg Ser Asp Lys Arg Trp Val Phe Val Asp Ala Met Lys Lys Lys
    50                  55                  60

His Ser Lys Val Gly Glu Met Leu Leu Gln Thr Phe Phe Ser Val Asp
65                  70                  75                  80

Pro Gly Ser His His Gly Glu Ala Asn Leu Glu Met Glu Glu Pro Glu
                85                  90                  95

Glu Ser Leu Asn Thr Leu Lys Leu Cys Ser Pro Glu Glu Phe Thr Arg
            100                 105                 110

Leu Cys Arg Glu Lys Thr Gln Glu Ile Tyr Pro Ile Lys Glu Ala Asn
        115                 120                 125

Gly Arg Thr Arg Lys Ala Leu Ile Ile Cys Asn Thr Glu Phe Lys His
    130                 135                 140

Leu Ser Leu Arg Tyr Gly Ala Asn Phe Asp Ile Ile Gly Met Lys Gly
145                 150                 155                 160

Leu Leu Glu Asp Leu Gly Tyr Asp Val Val Val Lys Glu Glu Leu Thr
                165                 170                 175

Ala Glu Gly Met Glu Ser Glu Met Lys Asp Phe Ala Ala Leu Ser Glu
            180                 185                 190

His Gln Thr Ser Asp Ser Thr Phe Leu Val Leu Met Ser His Gly Thr
        195                 200                 205

Leu His Gly Ile Cys Gly Thr Met His Ser Glu Lys Thr Pro Asp Val
    210                 215                 220

Leu Gln Tyr Asp Thr Ile Tyr Gln Ile Phe Asn Asn Cys His Cys Pro
225                 230                 235                 240

Gly Leu Arg Asp Lys Pro Lys Val Ile Ile Val Gln Ala Cys Arg Gly
                245                 250                 255

Gly Asn Ser Gly Glu Met Trp Ile Arg Glu Ser Ser Lys Pro Gln Leu
            260                 265                 270

Cys Arg Gly Val Asp Leu Pro Arg Asn Met Glu Ala Asp Ala Val Lys
        275                 280                 285

Leu Ser His Val Glu Lys Asp Phe Ile Ala Phe Tyr Ser Thr Thr Pro
    290                 295                 300

His His Leu Ser Tyr Arg Asp Lys Thr Gly Gly Ser Tyr Phe Ile Thr
305                 310                 315                 320

Arg Leu Ile Ser Cys Phe Arg Lys His Ala Cys Ser Cys His Leu Phe
                325                 330                 335

Asp Ile Phe Leu Lys Val Gln Gln Ser Phe Glu Lys Ala Ser Ile His
            340                 345                 350

Ser Gln Met Pro Thr Ile Asp Arg Ala Thr Leu Thr Arg Tyr Phe Tyr
        355                 360                 365

Leu Phe Pro Gly Asn
    370

<210>  7
<211>  1373
<212>  DNA
<213>  Homo sapiens

<400>  7
gggaggagag aaaagccatg gccgacaagg tcctgaagga gaagagaaag ctgtttatcc      60

gttccatggg tgaaggtaca ataaatggct tactggatga attattacag acaagggtgc     120

tgaacaagga agagatggag aaagtaaaac gtgaaaatgc tacagttatg gataagaccc     180

gagctttgat tgactccgtt attccgaaag gggcacaggc atgccaaatt tgcatcacat     240

acatttgtga agaagacagt tacctggcag ggacgctggg actctcagca gatcaaacat     300

ctggaaatta ccttaatatg caagactctc aaggagtact ttcttccttt ccagctcctc     360

aggcagtgca ggacaaccca gctatgccca catcctcagg ctcagaaggg aatgtcaagc 420
tttgctccct agaagaagct caaaggatat ggaaacaaaa gtcggcagag atttatccaa 480
taatggacaa gtcaagccgc acacgtcttg ctctcattat ctgcaatgaa gaatttgaca 540
gtattcctag aagaactgga gctgaggttg acatcacagg catgacaatg ctgctacaaa 600
atctggggta cagcgtagat gtgaaaaaaa atctcactgc ttcggacatg actacagagc 660
tggaggcatt tgcacaccgc ccagagcaca agacctctga cagcacgttc ctggtgttca 720
tgtctcatgg tattcgggaa ggcatttgtg ggaagaaaca ctctgagcaa gtcccagata 780
tactacaact caatgcaatc tttaacatgt tgaataccaa gaactgccca agtttgaagg 840
acaaaccgaa ggtgatcatc atccaggcct gccgtggtga cagccctggt gtggtgtggt 900
ttaaagattc agtaggagtt tctggaaacc tatctttacc aactacagaa gagtttgagg 960
atgatgctat taagaaagcc cacatagaga aggattttat cgctttctgc tcttccacac 1020
cagataatgt ttcttggaga catcccacaa tgggctctgt ttttattgga agactcattg 1080
aacatatgca agaatatgcc tgttcctgtg atgtggagga aattttccgc aaggttcgat 1140
tttcatttga gcagccagat ggtagagcgc agatgcccac cactgaaaga gtgactttga 1200
caagatgttt ctacctcttc ccaggacatt aaaataagga aactgtatga atgtctgtgg 1260
gcaggaagtg aagagatcgt tctgtaaaag gtttttggaa ttatgtctgc tgaataataa 1320
actttttttg aaataataaa tctggtagaa aaatgaaaaa aaaaaaaaaa aaa 1373

<210> 8
<211> 404
<212> PRT
<213> Homo sapiens

<400> 8

Met Ala Asp Lys Val Leu Lys Glu Lys Arg Lys Leu Phe Ile Arg Ser
1               5                   10                  15

Met Gly Glu Gly Thr Ile Asn Gly Leu Leu Asp Glu Leu Leu Gln Thr
            20                  25                  30

Arg Val Leu Asn Lys Glu Glu Met Glu Lys Val Lys Arg Glu Asn Ala
        35                  40                  45

Thr Val Met Asp Lys Thr Arg Ala Leu Ile Asp Ser Val Ile Pro Lys
    50                  55                  60

Gly Ala Gln Ala Cys Gln Ile Cys Ile Thr Tyr Ile Cys Glu Glu Asp
65                  70                  75                  80

Ser Tyr Leu Ala Gly Thr Leu Gly Leu Ser Ala Asp Gln Thr Ser Gly
                85                  90                  95

Asn Tyr Leu Asn Met Gln Asp Ser Gln Gly Val Leu Ser Ser Phe Pro
            100                 105                 110

Ala Pro Gln Ala Val Gln Asp Asn Pro Ala Met Pro Thr Ser Ser Gly
        115                 120                 125

Ser Glu Gly Asn Val Lys Leu Cys Ser Leu Glu Glu Ala Gln Arg Ile
    130                 135                 140

ttctgccctc gagcccaccg ggaacgaaag agaagctcta tctcgcctcc aggagcccag 60
ctatgaactc cttctccaca agcgccttcg gtccagttgc cttctccctg ggcctgctcc 120
tggtgttgcc tgctgccttc cctgccccag tacccccagg agaagattcc aaagatgtag 180
ccgccccaca cagacagcca ctcacctctt cagaacgaat tgacaaacaa attcggtaca 240
tcctcgacgg catctcagcc ctgagaaagg agacatgtaa caagagtaac atgtgtgaaa 300
gcagcaaaga ggcactggca gaaaacaacc tgaaccttcc aaagatggct gaaaaagatg 360
gatgcttcca atctggattc aatgaggaga cttgcctggt gaaaatcatc actggtcttt 420
tggagtttga ggtataccta gagtacctcc agaacagatt tgagagtagt gaggaacaag 480
ccagagctgt gcagatgagt acaaaagtcc tgatccagtt cctgcagaaa aaggcaaaga 540
atctagatgc aataaccacc cctgacccaa ccacaaatgc cagcctgctg acgaagctgc 600
aggcacagaa ccagtggctg caggacatga caactcatct cattctgcgc agctttaagg 660
agttcctgca gtccagcctg agggctcttc ggcaaatgta gcatgggcac ctcagattgt 720
tgttgttaat gggcattcct tcttctggtc agaaacctgt ccactgggca cagaacttat 780
gttgttctct atggagaact aaaagtatga gcgttaggac actattttaa ttatttttaa 840
tttattaata tttaaatatg tgaagctgag ttaatttatg taagtcatat ttatattttt 900
aagaagtacc acttgaaaca ttttatgtat tagttttgaa ataataatgg aaagtggcta 960
tgcagtttga atatcctttg tttcagagcc agatcatttc ttggaaagtg taggcttacc 1020
tcaaataaat ggctaactta tacatatttt taaagaaata tttatattgt atttatataa 1080
tgtataaatg gtttttatac caataaatgg cattttaaaa aattc 1125

<210> 10
<211> 212
<212> PRT
<213> Homo sapiens

<400> 10

Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala Pro Val Pro Pro
            20                  25                  30

Gly Glu Asp Ser Lys Asp Val Ala Ala Pro His Arg Gln Pro Leu Thr
        35                  40                  45

Ser Ser Glu Arg Ile Asp Lys Gln Ile Arg Tyr Ile Leu Asp Gly Ile
    50                  55                  60

Ser Ala Leu Arg Lys Glu Thr Cys Asn Lys Ser Asn Met Cys Glu Ser
65                  70                  75                  80

Ser Lys Glu Ala Leu Ala Glu Asn Asn Leu Asn Leu Pro Lys Met Ala
                85                  90                  95

Glu Lys Asp Gly Cys Phe Gln Ser Gly Phe Asn Glu Glu Thr Cys Leu
            100                 105                 110

Val Lys Ile Ile Thr Gly Leu Leu Glu Phe Glu Val Tyr Leu Glu Tyr
        115                 120                 125

Leu Gln Asn Arg Phe Glu Ser Ser Glu Glu Gln Ala Arg Ala Val Gln
    130                 135                 140

Met Ser Thr Lys Val Leu Ile Gln Phe Leu Gln Lys Lys Ala Lys Asn
145                 150                 155                 160

Leu Asp Ala Ile Thr Thr Pro Asp Pro Thr Thr Asn Ala Ser Leu Leu
                165                 170                 175

Thr Lys Leu Gln Ala Gln Asn Gln Trp Leu Gln Asp Met Thr Thr His
            180                 185                 190

Leu Ile Leu Arg Ser Phe Lys Glu Phe Leu Gln Ser Ser Leu Arg Ala
        195                 200                 205

Leu Arg Gln Met
    210